# EUROPEAN PATENT SPECIFICATION

(11) **EP 3 775 180 B1**
(45) Date of publication and mention of the grant of the patent: **12.01.2022**
(21) Application number: 19740536.8
(22) Date of filing: 12.07.2019
(51) Int. Cl.: C12N 9/02, C12Q 1/26

(54) **BIOSENSOR FOR DIAGNOSIS OF THYROID DYSFUNCTION**
BIOSENSOR ZUR DIAGNOSE VON FEHLFUNKTION DER SCHILDDRÜSE
BIOCAPTEUR POUR LE DIAGNOSTIC D'UN DYSFONCTIONNEMENT DE LA THYROÏDE

(30) Priority: 13.07.2018 EP 18183439
(43) Date of publication of application: 17.02.2021
(73) Proprietor: Aegirbio AB, 223 70 Lund (SE)
(72) Inventor: SRIVASTAVA, Sudha, 22381 Lund (SE); PUNYANI, Kushagr, 215 35 Malmö (SE); TAKWA, Mohamad, 22639 Lund (SE)
(74) Representative: Høiberg P/S
(86) International application number: PCT/EP2019/068869
(87) International publication number: WO 2020/012001

(56) References cited:
- WO-A2-03/025627
- WO-A2-2008/067007

## Description

### Technical field

The present invention relates to a biosensor and applications thereof for the quantification of free thyroid hormones to evaluate thyroid function.

### Background

The thyroid is a bilobed ductless gland situated in the front of the neck behind the Adam's apple. It is involved in synthesis and secretion of the iodine containing thyroid hormones - triiodothyronine (T3) and thyroxine (T4), which affect overall metabolic rate and protein synthesis. The secretion of thyroid hormones is governed by the hypothalamic-pituitary-thyroid axis (HPT axis), wherein the hypothalamus and pituitary glands stimulate the thyroid by releasing thyrotropin releasing hormone (TRH) and thyroid stimulating hormone (TSH).

T3, also known as triiodothyronine or [o-(4-Hydroxy-3,5-iodophenyl)3,5-diiodophenyl tyrosine] has an effect on increasing the basal metabolic rate, protein turnover, lipolysis, cardiac output, and fetal and infant development; while T4, also known as thyroxine or [o-(4-Hydroxy-3,5-diiodophenyl)3,5diiodophenyl tyrosine] is the prohormone that migrates to liver and kidneys, and serves as the substrate for site-specific synthesis of T3.

The thyroid hormones act on nearly every cell in the body. They act to increase the basal metabolic rate, affect protein synthesis, help regulate long bone growth (synergy with growth hormone) and neural maturation, and increase the body's sensitivity to catecholamines (such as adrenaline) by permissiveness. The thyroid hormones are essential to proper development and differentiation of all cells of the human body. These hormones also regulate protein, fat, and carbohydrate metabolism, affecting how human cells use energetic compounds. They also stimulate vitamin metabolism. Numerous physiological and pathological stimuli influence thyroid hormone synthesis.

Both excess and deficiency of thyroxine can cause disorders.
- Hyperthyroidism, which is often caused by Graves' Disease, clinical syndrome characterized by an excess of circulating free thyroxine (fT4), free triiodothyronine (fT3), or both, and reduced TSH. It is a common disorder that affects approximately 2% of women and 0.2% of men. Thyrotoxicosis is often used interchangeably with hyperthyroidism, but there are subtle differences. Although thyrotoxicosis also refers to an increase in circulating thyroid hormones, it can be caused by the intake of thyroxine tablets or by an over-active thyroid, whereas hyperthyroidism refers solely to an over-active thyroid.
- Hypothyroidism, which is often caused by Hashimoto's thyroiditis, is the case where there is a deficiency of thyroxine, triiodothyronine, or both.
- Clinical depression can sometimes be caused by hypothyroidism. T₃ is found in the junctions of synapses, and regulates the amounts and activity of serotonin, norepinephrine, and γ-aminobutyric acid (GABA) in the brain (Dratman M, Gordon J (1996). "Thyroid hormones as neurotransmitters". Thyroid. 6 (6): 639-47).
- Hair loss can sometimes be attributed to a malfunction of T₃ and T₄. Normal hair growth cycle may be affected disrupting the hair growth.
- Preterm births can suffer neurodevelopmental disorders due to lack of maternal thyroid hormones, at a time when their own thyroid is unable to meet their postnatal needs. Also in normal pregnancies, adequate levels of maternal thyroid hormone are vital in order to ensure thyroid hormone availability for the fetus and its developing brain. Congenital hypothyroidism occurs in every 1 in 1600-3400 newborns with most being born asymptomatic and developing related symptoms weeks after birth.

Hence, being able to quantify the amount of T3 and T4 in humans is important in the diagnosis of thyroid disorders. Conventional diagnosis of thyroid dysfunction is performed by immunoassays against the thyroid hormones and TSH. The current fT3 and fT4 tests attempt to competitively measure free hormones, or to measure after physical separation of the bound hormones, or by indirect estimation often being imprecise. Thus, there is impetus for finding new and alternative ways of quantifying free thyroid hormones.

A biosensor is a sensor that utilizes the molecule-identifying function of a biological material, e.g. a microorganism, enzyme, antibody, DNA, and RNA, and applies such a biological material as a molecule-identifying element. In other words, the biosensor utilizes the reaction occurring when an immobilized biological material identifies a target substrate, oxygen consumed by breathing of microorganism, enzyme reaction, luminescence, and the like. Among biosensors, practical use of enzyme sensors is developing. For example, enzyme sensors for glucose, lactic acid, uric acid, and amino acids find applications in medical instrumentation and food processing industry.

In an enzyme sensor, for example, electrons generated by the reaction of a substrate contained in a sample liquid, i.e. an analyte, with an enzyme or the like reduce an electron acceptor and a measuring device electrochemically measures the amount of the reduced electron acceptor. Thus, quantitative analysis of the analyte is performed. An example of such a biosensor is a sensor proposed in Patent Application No. PCT/JP00/08012.

Different techniques may be used to follow the reaction between for example an enzyme bound to an electrode and the target substrate. One of such techniques relies on Surface Plasmon Resonance (SPR). In SPR, one molecular partner such as a protein is immobilized on a metal (the chip). Light excites surface plasmons in the metal; when the binding partner binds to the immobilized molecule, this causes a detectable change in the surface plasmon signal. Another of such techniques relies on electrochemical transduction in which the content of a biological sample may be analyzed due to the direct conversion of a biological event to an electronic signal. The most common techniques in electrochemical biosensing comprise cyclic voltammetry, chronoamperometry, chronopotentiometry, impedance spectroscopy, and field-effect transistor based methods along with nanowire or magnetic nanoparticle-based biosensing.

WO 2008/067007 A2 discloses antibody-based measurements of thyroid hormone concentration by surface plasmon resonance. The measurement values obtained by this method are however not clinically significant as the method fails to discriminate between active (free) and inactive (bound) hormones.

### Summary

The present inventors found that the enzymes involved in conversion of thyroid hormones could be employed in a method for direct quantification of free T3 and free T4, by immobilization onto an electrode. These findings allow for a more precise and specific quantification of thyroid hormones as previous methods quantify free T3 and free T4 by multiple steps, indirect measurements or mere estimations. Hence, the present disclosure provides an enhanced diagnostic system for thyroid-related diseases.

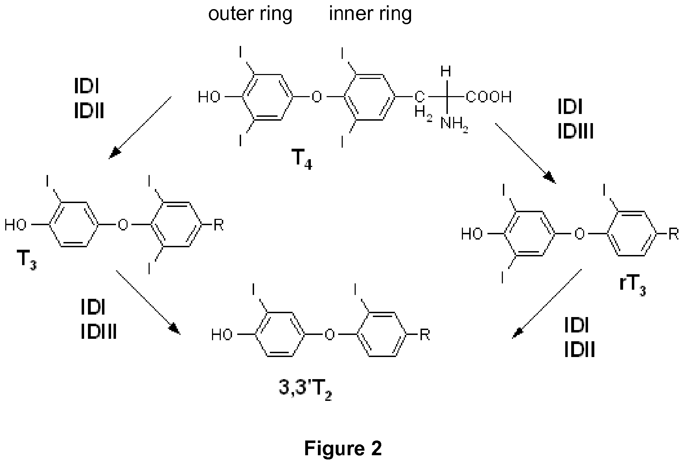

The present inventors have found that presence of at least two iodothyronine deiodinases selected from type I, type II and type III deiodinase together with an anti-rT3 antibody on the sensor, such as on separate surfaces of the substrate, is necessary for quantifying fT3 and/or fT4. In fact, one hormone cannot be quantified without quantifying the other hormone, because the enzymatic reactions used for quantification have some overlap, as can be seen in Fig. 2. Moreover, a third thyroid hormone, reverse triiodothyronine (rT3) plays an important role in the quantification, because deiodination of rT3 is also catalyzed by EC 1.21.99.4 iodothyronine deiodinases. Hence, rT3 may advantageously be removed or quantified separately prior to quantification of fT3 and fT4 with the sensor of the present invention. The sensor of the present invention comprises anti-rT3 antibodies that, together with the at least two iodothyronine deiodinases selected from type I, type II and type III deiodinase, allow specific quantification of rT3, fT3 and fT4 in one single step.

It is an aspect of the present disclosure to provide a sensor for quantification of a thyroid hormone, the sensor comprising
a. a substrate,
b. a combination of at least two iodothyronine deiodinases and an anti-rT3 antibody, immobilized on a surface of the substrate, wherein the combination comprises:
   i. type 1 iodothyronine deiodinase, type 2 iodothyronine deiodinase, and anti-rT3 antibody, or
   ii. type 2 iodothyronine deiodinase, type 3 iodothyronine deiodinase, and anti-rT3 antibody.

A further aspect of the present disclosure is to provide a method for quantification of a thyroid hormone in a sample, the method comprising the steps of:
a. Providing a sample comprising or suspected of comprising a thyroid hormone,
b. Contacting the sensor of the present disclosure,
c. Measuring a signal from the sensor, and
d. Using the signal to determine a level and/or concentration of the one or more thyroid hormones in the sample
thereby detecting the thyroid hormone.

A further aspect of the present disclosure is to provide an in-vitro method for diagnosis of a thyroid related disorder in a subject comprising the steps of:
a. Providing a sample obtained from the subject,
b. Determining a level and/or concentration of said thyroid hormone in the sample using the method of the present disclosure,
thereby diagnosing one or more thyroid related disorders.

It is a further aspect of the present disclosure to provide a method for manufacturing a sensor comprising at least two iodothyronine deiodinases, the method comprising:
a. providing a substrate,
b. providing a combination of the at least two iodothyronine deiodinases and an anti-rT3 antibody,
c. immobilizing the iodothyronine deiodinases and the anti-rT3 antibody on a surface of the substrate,
   wherein the combination comprises:
   - type 1 iodothyronine deiodinase, type 2 iodothyronine deiodinase, and anti-rT3 antibody, or
   - type 2 iodothyronine deiodinase, type 3 iodothyronine deiodinase, and anti-rT3 antibody,
thereby manufacturing a sensor comprising at least two iodothyronine deiodinases.

Another aspect of the present disclosure is to provide a hand-held device for quantification and/or monitoring of a thyroid hormone, the device comprising:
a. An inlet for a sample;
b. A sensor comprising:
   i. a substrate,
   ii. a combination of at least 2 iodothyronine deiodinases and an anti-rT3 antibody, wherein the combination comprises:
      - type 1 iodothyronine deiodinase, type 2 iodothyronine deiodinase, and anti-rT3 antibody, or
      - type 2 iodothyronine deiodinase, type 3 iodothyronine deiodinase, and anti-rT3 antibody;
c. A detector configured to receive a signal from the sensor and transform it into a format readable by a user;
d. Optionally, means for separating cellular components from the sample.

### Description of Drawings

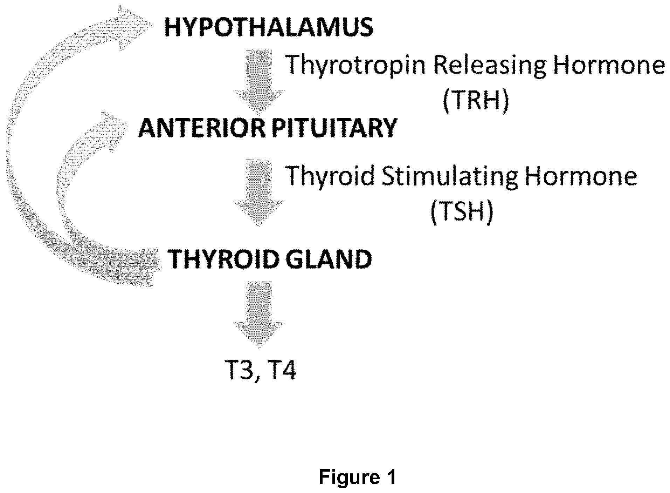

**Figure 1****.** The Hypothalamic-pituitary-thyroid axis.
**Figure 2****.** Catalysis of thyroid hormones by the three isoforms of iodothyronine deiodinases.
**Figure 3****.** Current response of the IDII amperometric biosensor with increasing concentrations of T4.
**Figure 4****.** Current response of the IDII voltammetric biosensor with increasing concentrations of T4.
**Figure 5****.** Effect of Thyroxine-binding globulin (TBG) concentration on detection of T4. The concentration of T4 is constant.
**Figure 6****.** Cyclic voltammetric measurements in fetal calf serum with increasing T4 concentrations.

### Detailed description

Disclosed herein is a biosensor and applications thereof for the quantification of free thyroid hormones fT3 and fT4 to evaluate thyroid function. Further, the present disclosure relates to methods for diagnosis or monitoring of thyroid related disorders in a subject comprising determining the concentration of a thyroid hormone in a sample obtained from said subject.

### Thyroid hormones

The present disclosure relates to a device and methods for detection and/or quantification of free thyroid hormones fT3 and fT4, and hence for evaluation of thyroid function.

Thyroid hormones are hormones produced and released by the thyroid gland. They are tyrosine-based hormones that are primarily responsible for regulation of metabolism.

The thyroid hormones thyroxine (T4) and triiodothyronine (T3) can be measured as *free thyroxine* (fT4) and *free triiodothyronine* (fT3), which are indicators of thyroxine and triiodothyronine activities in the body. They can also be measured as *total thyroxine* and *total triiodothyronine,* which will depend on the amount of thyroxine and triiodothyronine that is bound to thyroxine-binding globulin. A related parameter is the free thyroxine index, which is *total thyroxine* multiplied by thyroid hormone uptake, which, in turn, is a measure of the unbound thyroxine-binding globulins. Additionally, thyroid disorders can be detected prenatally using advanced imaging techniques and testing fetal hormone levels.

Reverse triiodothyronine (3,3',5'-triiodothyronine, reverse T3, or rT3) is an isomer of triiodothyronine (3,5,3' triiodothyronine, T3).

Reverse T3 is the third-most common iodothyronine the thyroid gland releases into the bloodstream, of which 0.9% is rT3; tetraiodothyronine (levothyroxine, T4) constitutes 90% and T3 is 9%. However, 95% of rT3 in human blood is made elsewhere in the body. The production of hormone by the thyroid gland is controlled by the hypothalamus and pituitary gland. The physiological activity of thyroid hormones is regulated by a system of enzymes that activate, inactivate or simply discard the prohormone T₄ and in turn functionally modify T₃ and rT₃. These enzymes operate under complex direction of systems including neurotransmitters, hormones, markers of metabolism and immunological signals. The levels of rT3 increase in conditions such as euthyroid sick syndrome because its clearance decreases while its production stays the same.

In healthy adult individuals, also referred to as healthy subjects, the reference intervals for the thyroid hormones are:
- fT3: 2.8-4.4 pg/mL (>= 1 year, retrieved from the Mayo Clinic),
- fT4: 0.8-2.0 ng/dL (all ages, retrieved from the Mayo Clinic),
- rT3: 10-24 ng/dL (retrieved from the Mayo Clinic),
as found in the following references: Demers LM, Spencer CI: The thyroid: pathophysiology and thyroid function testing. In Tietz Textbook of Clinical Chemistry and Molecular Diagnostics. Fourth edition. Edited by CA Burtis, ER Ashwood, DE Bruns. St. Louis, Elsevier Saunders Company. 2006, pp 2053-2087; Stockigt JR: Free thyroid hormone measurement. A critical appraisal. Clin Endocrinol Metab 2001 Jun;30:265-289; and Moore WT, Eastman RC: Diagnostic Endocrinology. St. Louis, Mosby, 1990, pp 182-183.

Different reference intervals may be used for infants and children. Moreover, different laboratories may adjust the reference intervals of ± 0.4 units.

Hence, a thyroid hormone concentration and/or level within the above intervals is perceived as normal, whereas a concentration and/or level below the intervals is considered low, and a concentration and/or level above is considered high.

### Iodothyronine deiodinases

The present disclosure relates to a sensor for quantification of a thyroid hormone, the sensor comprising a substrate, at least two iodothyronine deiodinases selected from EC 1.21.99.3 and/or EC 1.21.99.4, and an anti-rT3 antibody, wherein the at least two iodothyronine deiodinases and the anti-rT3 antibody are immobilized on a surface of the substrate.

The present disclosure relates to a sensor comprising an iodothyronine deiodinase [EC 1.21.99.3 and/or EC 1.21.99.4] or a fragment thereof, wherein the iodothyronine deiodinase is immobilized on the sensor, for detection and/or quantification of thyroid hormones.

lodothyronine deiodinases (EC 1.21.99.4 and EC 1.21.99.3) are a subfamily of deiodinase enzymes important in the activation and deactivation of thyroid hormones. Thyroxine (T4), the precursor of 3,5,3'-triiodothyronine (T3) is transformed into T3 by deiodinase activity. T3, through binding a nuclear thyroid hormone receptor, influences the expression of genes in practically every vertebrate cell. Iodothyronine deiodinases are unusual in that these enzymes contain selenium, in the form of an otherwise rare amino acid selenocysteine.

EC 1.21.99.4 iodothyronine deiodinases as referred to herein are enzymes capable of catalysing the following reaction:

3,5,3'-triiodo-L-thyronine + iodide + A + H(+) <=> L-thyroxine + AH(2)

EC 1.21.99.4 iodothyronine deiodinases have demonstrated enzymatic activity only in the direction of 5'-deiodination, which renders the thyroid hormone more active.

EC 1.21.99.4 iodothyronine deiodinases comprise of type I and type II enzymes, both containing selenocysteine, but with different kinetics. For the type I enzyme the first reaction is a reductive deiodination converting the -Se-H group of the enzyme into an - Se-I group; the reductant then reconverts this into -Se-H, releasing iodide. The following enzymes are comprised in the EC 1.21.99.4 group:
Diiodothyronine 5'-deiodinase;
lodothyronine 5'-deiodinase;
lodothyronine outer ring monodeiodinase;
L-thyroxine iodohydrolase (reducing);
Thyroxine 5-deiodinase;
Type I iodothyronine deiodinase;
Type II iodothyronine deiodinase.

EC 1.21.99.3 iodothyronine deiodinases as referred to herein are enzymes capable of catalysing the following reaction:

3,3',5'-triiodo-L-thyronine + iodide + acceptor + H(+) <=> L-thyroxine + reduced acceptor

EC 1.21.99.3 iodothyronine deiodinases have demonstrated enzymatic activity in the direction of 5-deiodination. This removal of the 5-iodine, i.e. from the inner ring, largely inactivates the hormone thyroxine. The following enzymes are comprised in the EC 1.21.99.4 group:
Diiodothyronine 5'-deiodinase;
lodothyronine 5-deiodinase;
lodothyronine inner ring monodeiodinase;
Type III iodothyronine deiodinase.

Type I deiodinase, also referred to as deiodinase type I (DI or D1) is commonly found in the liver and kidney and can deiodinate both the inner and the outer ring of the thyroid hormones. The terms "inner ring" and "outer ring" are visualised in figure 2 and refer to the different benzene rings present in the thyroid hormones.

Type II deiodinase, also referred to as deiodinase type II (DII or D2) is commonly found in the heart, skeletal muscle, central nervous system, fat, thyroid, and pituitary. It is only known to deiodinate the outer ring of the prohormone thyroxine and is the major activating enzyme (the already inactive reverse triiodothyronine is also degraded further by DII)

Type III deiodinase, also referred to as deiodinase type III (Dill or D3) is commonly found in the fetal tissue and the placenta; also present throughout the brain, except in the pituitary. It is only known to deiodinate the inner ring of thyroxine or triiodothyronine.

In tissues, deiodinases can either activate or inactivate thyroid hormones. Activation occurs by conversion of the prohormone thyroxine (T4) to the active hormone triiodothyronine (T3) through the removal of an iodine atom on the outer ring. Inactivation of thyroid hormones occurs by removal of an iodine atom on the inner ring, which converts thyroxine to the inactive reverse triiodothyronine (rT3), or which converts the active triiodothyronine to diiodothyronine (T2). The major part of thyroxine deiodination occurs within the cells.

DII activity can be regulated by ubiquitination: The covalent attachment of ubiquitin inactivates DII by disrupting dimerization and targets it to degradation in the proteosome. Deubiquitination removing ubiquitin from DII restores its activity and prevents proteosomal degradation.

DI both activates T4 to produce T3 and inactivates T4. Besides its increased function in producing extra thyroid T3 in patients with hyperthyroidism, its function is less well understood than DII or Dill. DII converts T4 into T3 and is a major source of the cytoplasmic T3 pool. Dill prevents T4 activation and inactivates T3.

DII and D3 are important in homeostatic regulation in maintaining T3 levels at the plasma and cellular levels. In hyperthyroidism, D2 is down regulated and D3 is upregulated to clear extra T3, while in hypothyroidism D2 is upregulated and D3 is downregulated to increase cytoplasmic T3 levels. Serum T3 levels remain fairly constant in healthy individuals, but D2 and D3 can regulate tissue specific intracellular levels of T3 to maintain homeostasis since T3 and T4 levels may vary by organ. Deiodinases also provide spatial and temporal developmental control of thyroid hormone levels. D3 levels are highest early in development and decrease over time, while D2 levels are high at moments of significant metamorphic change in tissues. Thus D2 enables production of sufficient T3 at necessary time points while D3 may shield tissue from overexposure to T3.

DII also plays a significant role in thermogenesis in brown adipose tissue (BAT). In response to sympathetic stimulation, dropping temperature, or overfeeding BAT, DII increases oxidation of fatty acids and uncouples oxidative phosphorylation via uncoupling protein, causing mitochondrial heat production. DII increases during cold stress in BAT and increases intracellular T3 levels. In DII deficient models, shivering is a behavioral adaptation to the cold. However, heat production is much less efficient than uncoupling lipid oxidation.

It is an aspect of the disclosure to provide a sensor for detection of a thyroid hormone, the sensor comprising an iodothyronine deiodinase [EC 1.21.99.3 and/or EC 1.21.99.4] or a fragment thereof, wherein the iodothyronine deiodinase is immobilized on the sensor.

In one embodiment, the iodothyronine deiodinase is type 1 iodothyronine deiodinase [EC 1.21.99.4], or a fragment thereof.

In one embodiment, the iodothyronine deiodinase is type 2 iodothyronine deiodinase [EC 1.21.99.4], or a fragment thereof.

In one embodiment, the iodothyronine deiodinase is type 3 iodothyronine deiodinase [EC 1.21.99.3], or a fragment thereof.

In one embodiment, the EC 1.21.99.4 iodothyronine deiodinase is type 2 iodothyronine deiodinase, or type 1 iodothyronine deiodinase.

In one embodiment, the EC 1.21.99.4 iodothyronine deiodinase is type 2 iodothyronine deiodinase.

In one embodiment, the EC 1.21.99.3 iodothyronine deiodinase is type 3 iodothyronine deiodinase.

In some embodiments of the present disclosure, the iodothyronine deiodinase is mammalian. In some embodiments of the present disclosure, the iodothyronine deiodinase is human.

In some embodiments of the present disclosure, the at least two iodothyronine deiodinase, selected from EC 1.21.99.3 and/or EC 1.21.99.4, and/or the anti-rT3 antibody are mammalian.

In some embodiments of the present disclosure, the at least two iodothyronine deiodinase, selected from EC 1.21.99.3 and/or EC 1.21.99.4, and/or the anti-rT3 antibody are human.

In some embodiments of the present disclosure, the at least two iodothyronine deiodinase, selected from EC 1.21.99.3 and/or EC 1.21.99.4 and/or the anti-rT3 antibody is conjugated to an additional moiety.

In some embodiments of the present disclosure, the at least two iodothyronine deiodinases, selected from EC 1.21.99.3 and/or EC 1.21.99.4, and/or the anti-rT3 antibody are each individually conjugated to an additional moiety. For example, the additional moiety may facilitate immobilization of the iodothyronine deiodinases on the sensor, or detection of the thyroid hormones.

In some embodiments of the present disclosure the substrate comprises multiple surfaces for immobilization of the at least two iodothyronine deiodinases, such as a first surface of the substrate and a second surface of the substrate.

In some embodiments of the present disclosure the first surface, the second surface and optionally the third surface is on the same substrate.

In some embodiments of the present disclosure the first surface, the second surface and optionally the third surface is on different substrates.

In some embodiments of the present disclosure the at least two iodothyronine deiodinases comprises a first iodothyronine deiodinase selected from EC 1.21.99.3 and/or EC 1.21.99.4 immobilized on the first surface of the substrate, and a second iodothyronine deiodinase selected from EC 1.21.99.3 and/or EC 1.21.99.4 immobilized on the second surface of the substrate.

In some embodiments of the present disclosure the first iodothyronine deiodinase is selected from EC 1.21.99.4 and the second iodothyronine deiodinase is selected from EC 1.21.99.3.

In some embodiments of the present disclosure the first iodothyronine deiodinase and the second iodothyronine deiodinase are both independently selected from EC 1.21.99.4.

In some embodiments of the present disclosure the first iodothyronine deiodinase and the second iodothyronine deiodinase are different enzymes selected from EC 1.21.99.4.

In some embodiments of the present disclosure the first iodothyronine deiodinase is type 1 iodothyronine deiodinase and/or type 2 iodothyronine deiodinase.

In some embodiments of the present disclosure the second iodothyronine deiodinase is type 1 iodothyronine deiodinase, type 2 iodothyronine deiodinase and/or type 3 iodothyronine deiodinase.

In some embodiments of the present disclosure the first iodothyronine deiodinase is a type 1 iodothyronine deiodinase and the second iodothyronine deiodinase is type 2 iodothyronine deiodinase.

In some embodiments of the present disclosure the first iodothyronine deiodinase is type 1 iodothyronine deiodinase and the second iodothyronine deiodinase is type 3 iodothyronine deiodinase.

In some embodiments of the present disclosure the first iodothyronine deiodinase is type 2 iodothyronine deiodinase and the second iodothyronine deiodinase is type 3 iodothyronine deiodinase.

In some embodiments, the additional moiety is a peptide, for example a polyhistidine tag (His-tag).

In some embodiments, the additional moiety is a label, also referred to as a fluorescent tag or a probe.

The polyhistidine-tag can be successfully used for the immobilization of proteins on a surface such as on a metal surface, for example a nickel- or cobalt-coated microtiter plate or on a protein array.

In some embodiments, the sensor according to the present disclosure comprises both type 2 iodothyronine deiodinase and type 3 iodothyronine deiodinase, or fragments thereof. The presence of both DII and DIII may result in a more precise diagnosis or thyroid disorders.

In some embodiments, the sensor according to the present disclosure comprises both type 1 iodothyronine deiodinase and type 2 iodothyronine deiodinase, or fragments thereof. The presence of both DI and DII may result in a more precise diagnosis or thyroid disorders.

In some embodiments, the sensor according to the present disclosure comprises both type 1 iodothyronine deiodinase and type 3 iodothyronine deiodinase, or fragments thereof. The presence of both DI and Dill may result in a more precise diagnosis or thyroid disorders.

In some embodiments, the sensor according to the present disclosure comprises a first iodothyronine deiodinase and a second iodothyronine deiodinase, both independently selected from EC 1.21.99.4.

In some embodiments, the sensor according to the present disclosure comprises a first iodothyronine deiodinase and a second iodothyronine deiodinase, both independently selected from EC 1.21.99.3.

In some embodiments, the sensor according to the present disclosure comprises a first and a second iodothyronine deiodinase both independently selected from EC 1.21.99.3 and EC 1.21.99.4.

In some embodiments, the sensor according to the present disclosure comprises a first iodothyronine deiodinase independently selected from EC 1.21.99.4 and a second iodothyronine deiodinase independently selected from EC 1.21.99.3 and EC 1.21.99.4.

In some embodiments, the sensor according to the present disclosure comprises the first iodothyronine deiodinase immobilized on a first surface of the substrate and the second iodothyronine deiodinase immobilized on a second surface of the substrate.

In some embodiments, the type 1 iodothyronine deiodinase comprises or consists of a polypeptide having at least 95% sequence identity, such as at least 96% sequence identity, such as at least 97% sequence identity, such as at least 98% sequence identity, such as at least 99% sequence identity entity, such as about 100% sequence identity to SEQ ID NO: 1, or a fragment thereof.

In some embodiments, the type 2 iodothyronine deiodinase comprises or consists of a polypeptide having at least 95% sequence identity, such as at least 96% sequence identity, such as at least 97% sequence identity, such as at least 98% sequence identity, such as at least 99% sequence identity entity, such as about 100% sequence identity to SEQ ID NO: 2, or a fragment thereof.

In some embodiments, the type 3 iodothyronine deiodinase comprises or consists of a polypeptide having at least 95% sequence identity, such as at least 96% sequence identity, such as at least 97% sequence identity, such as at least 98% sequence identity, such as at least 99% sequence identity entity, such as about 100% sequence identity to SEQ ID NO: 3, or a fragment thereof.

In some embodiments, the iodothyronine deiodinase is recombinantly produced, such as by means of cell-free expression.

In some embodiments, the EC 1.21.99.3 iodothyronine deiodinase, the EC 1.21.99.4 iodothyronine deiodinase and/or the anti-rT3 antibody are recombinantly produced, such as by means of cell-free expression.

Cell-free expression, also referred to as cell-free protein synthesis or CFPS, is the production of protein using biological machinery in a cell-free system, that is, without the use of living cells. The in vitro protein synthesis environment is not constrained by a cell wall or homeostasis conditions necessary to maintain cell viability. Thus, CFPS enables direct access and control of the translation environment which is advantageous for a number of applications including co-translational solubilisation of membrane proteins, optimisation of protein production, incorporation of non-natural amino acids, selective and site-specific labelling.

In reference to sequence identity: a high level of sequence identity indicates likelihood that the first sequence is derived from the second sequence. Amino acid sequence identity requires identical amino acid sequences between two aligned sequences. Thus, a candidate sequence sharing at least 95% amino acid identity with a reference sequence, requires that, following alignment, at least 95% of the amino acids in the candidate sequence are identical to the corresponding amino acids in the reference sequence. Identity may be determined by aid of computer analysis, such as, without limitations, the ClustalW computer alignment program (Higgins D., Thompson J., Gibson T., Thompson J.D., Higgins D.G., Gibson T.J., 1994. CLUSTAL W: improving the sensitivity of progressive multiple sequence alignment through sequence weighting, position-specific gap penalties and weight matrix choice. Nucleic Acids Res. 22:4673-4680), and the default parameters suggested therein.

### Biosensor concept

The present disclosure relates to a biosensor, that is a sensor comprising a substrate, at least two iodothyronine deiodinases selected from EC 1.21.99.3 and/or EC 1.21.99.4, and an anti-rT3 antibody, wherein the at least two iodothyronine deiodinases and the anti-rT3 antibody are immobilized on a surface of the substrate.

The present disclosure relates to a biosensor, that is a sensor comprising an iodothyronine deiodinase [EC 1.21.99.3 and/or EC 1.21.99.4] immobilized on one of its surfaces.

A variety of devices for detecting ligand/receptor interactions are known. The most basic of these are purely chemical/enzymatic assays in which the presence or amount of analyte is detected by measuring or quantitating a detectable reaction product. Ligand/receptor interactions can also be detected and quantitated by radiolabel assays.

Quantitative binding assays of this type involve two separate components: a reaction substrate, e.g., a solid-phase test strip, a chip or an electrode, and a separate reader or detector device, such as a scintillation counter or spectrophotometer. The substrate is generally unsuited to multiple assays, or to miniaturization, for handling multiple analyte assays from a small amount of body-fluid sample.

In biosensors, by contrast, the assay substrate and detector surface are integrated into a single device. One general type of biosensor employs an electrode surface in combination with current or impedance measuring elements for detecting a change in current or impedance in response to the presence of a ligand-receptor binding event. Another type of biosensor may employ a chip, for example a glass chip, in combination with an optical detector, for example in combination with surface plasmon resonance.

A "biosensor", sometimes referred to as "sensor" herein refers to a system comprising a sensor and a biological element. Biosensors are practically substitutes of conventional analytical techniques that are tedious, costly, complex and not appropriate for in situ supervising. A biosensor is a chemical analytical device unifying a biological element with a transducer adumbratively. It consolidates a biological element within or in intimate contact with a transducer which yields an electronic signal proportional to a single analyte that is further conveyed to a detector.

A biosensor embraces three fundamental components that are a bioreceptor (the biological element), a transducer and an electronic circuit. The bioreceptor or biological element is a biomolecule that is embedded with the transducer, like an enzyme, DNA, protein, whole cell, antibodies etc. In the present application, the bioreceptor is an iodothyronine deiodinase. The transducer is a device that renovates one form of energy into another, like chemical energy into electrical energy. For example, the transducer is a detector. Detectors encompassed by the methods of the present disclosure are optical detectors, such as a surface plasmon resonance detector, electrochemical detectors, and measurement circuits. Electronic circuit comprises a signal processing system that converts an electrical signal into a processable signal.

Biosensors based on surface plasmon resonance (SPR) effects exploit the shift in SPR surface reflection angle that occurs with perturbations, e.g., binding events, at the SPR interface. Finally, biosensors may also utilize changes in optical properties at a biosensor surface.

Electrochemical biosensors are normally based on enzymatic catalysis of a reaction that produces or consumes electrons (redox enzymes). The sensor substrate usually contains three electrodes; a reference electrode, a working electrode and a counter electrode. The target analyte is involved in the reaction that takes place on the active electrode surface, and the reaction may cause either electron transfer across the double layer (producing a current) or can contribute to the double layer potential (producing a voltage). Either the current can be measured, wherein the rate of flow of electrons is proportional to the analyte concentration at a fixed potential or the potential can be measured at zero current, which gives a logarithmic response. Further, the label-free and direct electrical detection of small peptides and proteins is possible by their intrinsic charges using biofunctionalized ion-sensitive field-effect transistors.

Potentiometric biosensors, in which potential is produced at zero current, gives a logarithmic response with a high dynamic range. Such biosensors are often made by screen printing the electrode patterns on a plastic substrate, coated with a conducting polymer and then some protein (enzyme or antibody) is attached. They have only two electrodes and are extremely sensitive and robust. They enable the detection of analytes at levels previously only achievable by HPLC and LC/MS and without rigorous sample preparation. All biosensors usually involve minimal sample preparation as the biological sensing component is highly selective for the analyte concerned. The signal is produced by electrochemical and physical changes in the conducting polymer layer due to changes occurring at the surface of the sensor. Such changes can be attributed to ionic strength, pH, hydration and redox reactions. Field effect transistors (FET), in which the gate region has been modified with an enzyme or antibody, can also detect very low concentrations of various analytes as the binding of the analyte to the gate region of the FET cause a change in the drain-source current.

Biosensors have a number of potential advantages over binding assay systems having separate reaction substrates and reader devices. One important advantage is the ability to manufacture small-scale, but highly reproducible, biosensor units using microchip manufacturing methods.

There are many potential applications of biosensors of various types. The main requirements for a biosensor approach to be valuable in terms of research and commercial applications are the identification of a target molecule, availability of a suitable biological recognition element, and the potential for disposable portable detection systems to be preferred to sensitive laboratory-based techniques in some situations.

In some embodiments, the present disclosure relates to a biosensor for detection and/or quantification of a thyroid hormone, wherein the thyroid hormone is selected from free T4, free T3 and reverse T3 (rT3).

In some embodiments, the present disclosure relates to a biosensor for quantification of a thyroid hormone, wherein the thyroid hormone is selected from free T4, free T3 and reverse T3 (rT3).

In some embodiments, the present disclosure relates to a biosensor, wherein said biosensor comprises a sensor, said sensor comprising a substrate,
at least two iodothyronine deiodinases selected from EC 1.21.99.3 and/or EC 1.21.99.4, or a fragment thereof, and an anti-rT3 antibody, or a fragment thereof, wherein the at least two iodothyronine deiodinases and the anti-rT3 antibody are immobilized on a surface of the substrate.

In some embodiments, the present disclosure relates to a biosensor, wherein said biosensor comprises a sensor, said sensor comprising an electrode and an iodothyronine deiodinase [EC 1.21.99.3 and/or EC 1.21.99.4] or a fragment thereof immobilized on the electrode.

In some embodiments, the sensor according to the present disclosure comprises between 10 and 100 IU of an iodothyronine deiodinase, such as between 10 and 15 IU, such as between 15 and 20 IU, such as between 20 and 25 IU, such as between 25 and 30 IU, such as between 30 and 35 IU, such as between 35 and 40 IU, such as between 40 and 45 IU, such as between 45 and 50 IU, such as between 50 and 55 IU, such as between 55 and 60 IU, such as between 60 and 65 IU, such as between 65 and 70 IU, such as between 70 and 75 IU, such as between 75 and 80 IU, such as between 80 and 85 IU, such as between 85 and 90 IU, such as between 90 and 95 IU, such as between 95 and 100 IU.

In some embodiments, the sensor according to the present disclosure comprises between 10 and 100 IU of EC 1.21.99.3 iodothyronine deiodinase and EC 1.21.99.4 iodothyronine deiodinase, such as between 10 and 15 IU, such as between 15 and 20 IU, such as between 20 and 25 IU, such as between 25 and 30 IU, such as between 30 and 35 IU, such as between 35 and 40 IU, such as between 40 and 45 IU, such as between 45 and 50 IU, such as between 50 and 55 IU, such as between 55 and 60 IU, such as between 60 and 65 IU, such as between 65 and 70 IU, such as between 70 and 75 IU, such as between 75 and 80 IU, such as between 80 and 85 IU, such as between 85 and 90 IU, such as between 90 and 95 IU, such as between 95 and 100 IU.

In some embodiments, the sensor according to the present disclosure comprises between 10 and 100 IU of type 2 and the type 3 iodothyronine deiodinases, such as between 10 and 15 IU, such as between 15 and 20 IU, such as between 20 and 25 IU, such as between 25 and 30 IU, such as between 30 and 35 IU, such as between 35 and 40 IU, such as between 40 and 45 IU, such as between 45 and 50 IU, such as between 50 and 55 IU, such as between 55 and 60 IU, such as between 60 and 65 IU, such as between 65 and 70 IU, such as between 70 and 75 IU, such as between 75 and 80 IU, such as between 80 and 85 IU, such as between 85 and 90 IU, such as between 90 and 95 IU, such as between 95 and 100 IU.

IU stays for international unit, and it is a unit of measurement for the amount of a substance; the mass or volume that constitutes one international unit varies based on which substance is being measured, and the variance is based on the biological activity or effect, for the purpose of easier comparison across substances.

In some embodiments of the present disclosure, it is disclosed a sensor for quantification of a thyroid hormone comprising:
- a first electrode comprising a first surface;
- a second electrode comprising a second surface;
- a first iodothyronine deiodinase selected from EC 1.21.99.4 immobilized on the first surface of the first electrode;
- a second iodothyronine deiodinase selected from EC 1.21.99.3 immobilized on the second surface of the second electrode.

In some embodiments of the present disclosure, it is disclosed a sensor for quantification of a thyroid hormone comprising:
- a first electrode comprising a first surface;
- a second electrode comprising a second surface;
- a third electrode comprising a third surface;
- a first iodothyronine deiodinase selected from EC 1.21.99.4 immobilized on the first surface of the first electrode;
- a second iodothyronine deiodinase selected from EC 1.21.99.3 and EC 1.21.99.3 immobilized on the second surface of the second electrode, and
- an anti-rT3 antibody immobilized on the third surface of the third electrode.

It is an aspect of the present disclosure to provide a method for manufacturing a sensor comprising an iodothyronine deiodinase, the method comprising:
a) Providing an electrode,
b) Providing a combination of the at least two iodothyronine deiodinases and an anti-rT3 antibody,
c) immobilizing the iodothyronine deiodinases and the anti-rT3 antibody on a surface of the substrate, wherein the combination comprises:
   a. type 1 iodothyronine deiodinase, type 2 iodothyronine deiodinase, and anti-rT3 antibody, or
   b. type 2 iodothyronine deiodinase, type 3 iodothyronine deiodinase, and anti-rT3 antibody,,
   thereby manufacturing a sensor comprising the iodothyronine deiodinase.

In a particular embodiment, the electrode is as defined according to the embodiments of the present disclosure. In a particular embodiment, the iodothyronine deiodinase is as defined according to the embodiments of the present disclosure.

Step c) above comprises immobilization of an iodothyronine deiodinase on the electrode, such as on the sensor. The step may be seen as comprising a step of functionalizing the electrode, followed by immobilization of the iodothyronine deiodinase on the functionalized electrode. Examples of procedures that may be used to immobilize an iodothyronine deiodinase on an electrode are described in detail herein in the present disclosure.

### Enzyme immobilization

Immobilization of the biological element, such as an enzyme of interest on the surface of the sensor (be it metal, polymer or glass) is a necessary and critical step in the design of biosensors. Different immobilization techniques exist depending on the substrate employed, these techniques are known to the person skilled in the art.

It is an aspect of the disclosure to provide a sensor for detection of a thyroid hormone, the sensor comprising a substrate,
a. a combination of at least two iodothyronine deiodinases and an anti-rT3 antibody, immobilized on a surface of the substrate, wherein the combination comprises:
   - type 1 iodothyronine deiodinase, type 2 iodothyronine deiodinase, and anti-rT3 antibody, or
   - type 2 iodothyronine deiodinase, type 3 iodothyronine deiodinase, and anti-rT3 antibody,
wherein the first iodothyronine deiodinase, the second iodothyronine deiodinase and the anti-rT3 antibody are immobilized on a surface of the substrate.

In some embodiments, the substrate according to the present disclosure comprises one or more electrodes and/or chips.

In some embodiments, the substrate according to the present disclosure comprises at least 1 electrode, such as at least 2 electrodes, such as at least 3 electrodes.

In some embodiments, the substrate according to the present disclosure comprises at least 1 chip, such as at least 2 chips, such as at least 3 chips.

In some embodiments, the substrate according to the present disclosure comprises or consists of 3 electrodes, and wherein the first surface is a surface of a first electrode, the second surface is a surface of a second electrode, and the third surface is a surface of a third electrode.

In some embodiments, the substrate according to the present disclosure comprises or consists of three chips, and wherein the first surface is a surface of a first chip, the second surface is a surface of a second chip, and the third surface is a surface of a third chip.

In some embodiments, the sensor according to the present disclosure comprises a substrate having a modified surface. Said substrate is modified so that an iodothyronine deiodinase may be immobilized on its surface.

In some embodiments, the substrate according to the present disclosure is an electrode or a chip. In further embodiments, said chip is a glass chip. The term "glass" as used herein is equivalent to quartz or silica, comprising silicon and oxygen atoms in a continuous framework with an overall chemical formula of SiO₂.

In some embodiments, the sensor according to the present disclosure comprises one electrode, such as two electrodes, such as three electrodes, wherein the first surface of the first electrode is a modified surface, wherein the second surface of the second electrode is a modified surface, and/or wherein the third surface of the third electrode is a modified surface.

When referring to "electrode" herein in the present disclosure it is referred to a first electrode, a second electrode, a third electrode and/or a further electrode on which the biocomponents of the sensor, that is the first iodothyronine deiodinase selected from EC 1.21.99.4, the second iodothyronine deiodinase selected form EC 1.21.99.3 and EC 1.21.99.4, and the anti-rT3 antibody are immobilized.

When referring to "surface" herein in the present disclosure it is referred to a first surface, a second surface, a third surface and/or a further surface of the substrates (that is of the electrodes and/or of the chips) on which the biocomponents of the sensor, that is the first iodothyronine deiodinase selected from EC 1.21.99.4, the second iodothyronine deiodinase selected from EC 1.21.99.3 and EC 1.21.99.4, and the anti-rT3 antibody are immobilized.

In some embodiments, the electrode is made of carbon, gold or platinum.

In a further embodiment, the electrode is a screen printed electrode.

In some embodiments, the sensor according to the present disclosure comprises at least a surface of the chip or of the electrode coated with a layer or monolayer of gold. In further embodiments, the surface of the chip or of the electrode is coated with a material selected from the group consisting of silver, copper oxide, graphene, iron oxide and a combination thereof.

In some embodiments according to the present disclosure the first, second and/or third surface of the substrate of the sensor is a modified surface.

The sensor surfaces, for example the electrodes' surfaces and/or the chips' surfaces) may be subjected to any type of treatment before use. The treatment may include deposition of nanoparticles which may be carried out by methods such as coating, dip coating, spin coating, Langmuir-Blodgett, self-assembly, solvent evaporation, doctor blade coating, chemical vapor deposition, transfer printing, direct deposition, deposition-precipitation method, use of a sticking layer between electrode surface and nanoparticle containing polyelectrolytes, covalent immobilization, such as by an amide bond, electrostatic immobilization, polymer brush immobilization, sol-gel/polymer network immobilization, van der Waals immobilization, hydrophobic/hydrophilic immobilization, deposition by evaporation and/or dewetting, electrodeposition such as optically induced electrodeposition, Turkevich-Frens method, Brust-Schiffrin method, layer-by-layer, successive ionic layer deposition, chemical methods, photochemical methods, sonochemical methods or a combination thereof.

The surface of the sensors may further be patterned, such as by microfabrication techniques, prior to deposition of nanoparticles, such that at least a part of the nanoparticles are immobilized in a specific pattern on the surface of the sensors. The surfaces of the sensors may further be treated to induce chemical changes, such as functionalization or activation by for example amine functionalization, thiol functionalization, hydroxylation, silanization, oxidizing and/or plasma activation or a combination thereof. The surface modification may be performed at any point in the treatment of the surfaces, such as before deposition of nanoparticles.

The sensors may further be assembled or treated according to any method available for the assembly of sensors, such as by sintering, printing, such as 3d printing, screen printing and/or ink-jet printing, casting, electrodeposition, thin film technology, network formation, dealloying, lithography such as optical lithography and/or imprint lithography, sputtering, stamping, thermal annealing, electrolysis, anodization, etching, such as electrochemical etching, wet etching and dry etching or a combination thereof.

In some embodiments according to the present disclosure, the modified surface is a surface comprising a plurality of topographic features in the nanometre and/or micrometre size. Such a modified surface comprising a plurality a topographic features in the nanometre and/or micrometre size is also referred to as a roughened surface, or a rough surface. A roughened surface is beneficial for immobilization of enzymes because it minimizes Van der Waals forces which may otherwise cause the immobilized enzymes to collapse.

In some embodiments according to the present disclosure, the plurality of topographic features in the nanometre and/or micrometre size are selected from the group consisting of: microparticles, nanoparticles, microwires, nanowires, microtubes, nanotubes, microrods, nanorods, and combinations thereof.

In some embodiments according to the present disclosure, the plurality of topographic features in the nanometre and/or micrometre size have been generated on the surface of the substrate by assembling said surface by sintering.

In some embodiments according to the present disclosure, wherein the plurality of topographic features in the nanometre and/or micrometre size have been generated on the surface of the substrate by surface etching. For example, surface etching may be wet etching or dry etching.

In some embodiments according to the present disclosure, wherein the plurality of topographic features in the nanometre and/or micrometre size have been generated on the surface of the substrate by particle deposition. For example, the plurality of topographic features in the nanometre and/or micrometre size may have been generated on the surface of the substrate by electrophoretic deposition.

In some embodiments according to the present disclosure, wherein the modified surface is a surface coated with a layer of gold.

In some embodiments according to the present disclosure, wherein the modified surface is a surface coated with nanoparticles selected from the group consisting of gold, silver, copper oxide, graphene, iron oxide and combinations thereof.

In some embodiments, the sensor according to the present disclosure comprises at least a surface of the substrate (chip or electrode) coated with a layer or monolayer of gold, and said surface is further modified with nanoparticles selected from the group consisting of gold, silver, copper oxide, graphene, iron oxide and combinations thereof.

In other words, the substrate (electrode or chip) may be coated with a layer or monolayer of gold, on said layer or monolayer of gold there may be nanoparticles, for examples nanoparticles selected from the group consisting of gold, silver, copper oxide, graphene, iron oxide and combinations thereof, and the iodothyronine deiodinase molecules may be immobilized on said nanoparticles.

In one embodiment, the at least one surface of the chip or of the electrode is modified with nanoparticles selected from the group consisting of gold, silver, copper oxide, graphene, iron oxide and combinations thereof.

In some embodiments according to the present disclosure, wherein the modified surface is a surface coated with a layer of gold, and wherein said surface is further modified with nanoparticles selected from the group consisting of gold, silver, copper oxide, graphene, iron oxide and combinations thereof.

The nanoparticles as disclosed herein may be capped with a capping agent. A capping agent may be organic molecules, such as citrate, and may be used to stop the growth of the nanoparticle to control its size. In some embodiments, the nanoparticles are citrate-capped, amino-capped or both citrate- and amino-capped.

In one embodiment, the sensor comprises a chip having a modified surface, wherein said chip is a chemically modified glass substrate, and wherein said chip is used in combination with surface plasmon resonance for detecting and/or quantifying a thyroid hormone.

In one embodiment, the sensor comprises a chip having a modified surface, wherein said chip is a glass substrate modified with nanoparticles, wherein said nanoparticles may be selected from the group consisting of gold, silver, copper oxide, graphene, iron oxide and combinations thereof, and wherein said chip is used in combination with surface plasmon resonance for detecting and/or quantifying a thyroid hormone.

In one embodiment, the sensor comprises a chip having a modified surface, wherein said chip is a glass substrate modified with a layer or monolayer, wherein said layer or monolayer is made of a material selected from the group consisting of gold, silver, copper oxide, graphene, iron oxide and combinations thereof, and wherein said chip is used in combination with surface plasmon resonance for detecting and/or quantifying a thyroid hormone.

In one embodiment, the sensor comprises a chip having a modified surface, wherein said chip is a glass substrate modified with gold nanoparticles, and wherein said chip is used in combination with surface plasmon resonance for detecting and/or quantifying a thyroid hormone.

In one embodiment, the sensor comprises a chip having a modified surface, wherein said chip comprises a glass substrate modified with a gold layer or monolayer, and wherein said chip is used in combination with surface plasmon resonance for detecting and/or quantifying a thyroid hormone.

In one embodiment, the sensor comprises an electrode having a modified surface, wherein said electrode comprises a layer or monolayer surface made of gold, and wherein said electrode is used in combination with electrochemical transduction for detecting and/or quantifying a thyroid hormone.

In another embodiment, the electrode comprises a layer or monolayer surface made of silver, wherein said electrode is used in combination with electrochemical transduction for detecting and/or quantifying a thyroid hormone according to the present disclosure.

In another embodiment, the electrode comprises a layer or monolayer surface made of copper oxide, wherein said electrode is used in combination with electrochemical transduction for detecting and/or quantifying a thyroid hormone according to the present disclosure.

In another embodiment, the electrode comprises a layer or monolayer surface made of graphene, wherein said electrode is used in combination with electrochemical transduction for detecting and/or quantifying a thyroid hormone according to the present disclosure.

In another embodiment, the electrode comprises a layer or monolayer surface made of iron oxide, wherein said electrode is used in combination with electrochemical transduction for detecting and/or quantifying a thyroid hormone according to the present disclosure.

In another embodiment, the electrode comprises a layer or monolayer surface made of a combination of metals, such as gold and silver, wherein said electrode is used in combination with electrochemical transduction for detecting and/or quantifying a thyroid hormone according to the present disclosure.

In some embodiments, the iodothyronine deiodinase according to the present disclosure is immobilized on the substrate.

In some embodiments, the iodothyronine deiodinases according to the present disclosure are immobilized on the surface of the sensor through a linker comprising a nanoparticle.

In some embodiments, the iodothyronine deiodinases according to the present disclosure are immobilized on the surface of the sensor through a linker comprising a nickel-histidine (Ni-His) covalent coordinate bond. This may be particularly suitable when the iodothyronine deiodinases comprise an histidine-tag.

In some embodiments according to the present disclosure, wherein the nanoparticle has a size of between 1 nm and 50 nm, preferably a size of between 5 nm and 45 nm, preferably a size of between 10 nm and 40 nm, preferably a size of between 10 nm and 35 nm, preferably a size of between 10 nm and 30 nm. The size of the nanoparticles may be determined with TEM microscopy. Use of nanoparticles in this size range as linkers between the surface of the substrate and the iodothyronine deiodinases may give the surface of the substrate a preferred curvature for immobilization of the iodothyronine deiodinases.

In some embodiments, the iodothyronine deiodinase according to the present disclosure is immobilized on the substrate via ionic interactions.

In some embodiments, the iodothyronine deiodinase according to the present disclosure is immobilized on the substrate via non-covalent interactions.

In some embodiments, the iodothyronine deiodinase according to the present disclosure is covalently immobilized on the substrate.

In some embodiments, the iodothyronine deiodinase is immobilized on the substrate through a linker comprising one or more nanoparticles. The presence of at least one nanoparticle between the substrate and the iodothyronine deiodinase prevents unfolding of the protein.

In some embodiments, the iodothyronine deiodinase is immobilized on the substrate through a linker comprising:
a cysteamine bound to the electrode, and
a nanoparticle bound to the cysteamine and to the iodothyronine deiodinase, optionally through one or more additional cysteamines.

In some embodiments, the at least two iodothyronine deiodinases and/or the anti-rT3 antibody, are immobilized on the substrate through a linker comprising:
a cysteamine bound to the electrode, and
a nanoparticle bound to the cysteamine and to the iodothyronine deiodinase, optionally through one or more additional cysteamines.

In some embodiments, the sensor of the present disclosure further comprises anti-rT3 antibody immobilized on a surface of the sensor, in particular immobilized on a surface of an electrode (for example on the third surface of the third electrode) or on a surface of a chip (for example on the third surface of the third chip).

Various techniques useful in immobilization of antibodies on a surface of an electrode or on a surface of a chip are known to the person of skills in the art.

In some embodiments of the present disclosure, anti-rT3 antibody is immobilized on the surface of the substrate via 1-ethyl-3-(3-dimethylaminopropyl)carbodiimide hydrochloride (EDC) - N-hydroxysuccinimide (NHS) chemistry.

In some embodiments of the present disclosure, anti-rT3 antibody may be an azide-modified antibody and it may be immobilized on the surface of the substrate via click chemistry on alkyne or heavy chain-associated glycans.

In some embodiments of the present disclosure, anti-rT3 antibody is immobilized on the surface of the substrate directly, wherein the surface of the substrate is a positively charged amine-modified surface.

In some embodiments of the present disclosure, anti-rT3 antibody is immobilized on the surface of the substrate via biotin-avidin binding, in such a case the anti-rT3 antibody is biotinylated.

In some embodiments, the iodothyronine deiodinase is bound via the C-terminal or the N-terminal to the linker. In some embodiments, the iodothyronine deiodinase is bound through the N-terminal to the linker by an amide bond with cysteamine.

For example, the iodothyronine deiodinase may be immobilized on a surface of the substrate by employing any of the following procedures:
- Carbon electrodes: The electrodes can be oxidized using H₂SO₄/HNO₃ (aq) to introduce hydroxyl groups; washing; introduction of free thiol groups via reaction with Cysteamine Hydrochloride (aq) in dark; washing; Covalent immobilization of citrate-capped nanoparticles using thiol/gold coupling; washing; introduction of free amine groups using Cysteamine Dihydrochloride (aq); washing; immobilization of an iodothyronine deiodinase or fragment thereof via amide bond formation through their carboxylate residues; washing; blocking with Bovine Serum Albumin. See also Sharma S, Zapatero-Rodriguez J, Saxena R, O'Kennedy R, Srivastava S 2018. Ultrasensitive direct impedimetric immunosensor for detection of serum HER2. Biosensors & Bioelectronics 106:78-85.
- Gold electrodes: Introduction of amino groups by reaction with Cysteamine Hydrochloride (aq) exploiting Au/SH coupling; washing; covalent attachment of citrate-capped nanoparticles by formation of an amide bond; washing; immobilization of an iodothyronine deiodinase or fragment thereof by covalent coupling between their primary amine and the free carboxylate residues at the nanoparticles. See also Raghav R, Srivastava S, 2016. Immobilization Strategy for Enhancing Sensitivity of Immunosensors: L-Asparagine-AuNPs as a promising alternative of EDC-NHS activated citrate-AuNPs for Antibody immobilization. Biosensors & Bioelectronics 15;78:396-403..
- Gold electrodes: Introduction of amino groups by reaction with Cysteamine Hydrochloride (aq) exploiting Au/SH coupling; washing; covalent attachment of citrate-capped nanoparticles by formation of an amide bond; washing; addition of aqueous solution of EDC crosslinker (1-ethyl-3-(3-dimethylaminopropyl)carbodiimide hydrochloride) and Sulfo-NHS (N-hydroxysulfosuccinimide); immobilization of an iodothyronine deiodinase or fragment thereof by covalent coupling between their primary amine and the NHS ester intermediate. See also Raghav R, Srivastava S, 2016. Immobilization Strategy for Enhancing Sensitivity of Immunosensors: L-Asparagine-AuNPs as a promising alternative of EDC-NHS activated citrate-AuNPs for Antibody immobilization. Biosensors & Bioelectronics 15;78:396-403..
- Gold electrodes: Introduction of amino groups by reaction with Cysteamine Hydrochloride (aq) exploiting Au/SH coupling; washing; covalent attachment of amino and citrate-capped nanoparticles by formation of an amide bond; washing; immobilization of an iodothyronine deiodinase or fragment thereof by covalent coupling by formation of amide bonds . See also Raghav R, Srivastava S, 2016. Immobilization Strategy for Enhancing Sensitivity of Immunosensors: L-Asparagine-AuNPs as a promising alternative of EDC-NHS activated citrate-AuNPs for Antibody immobilization. Biosensors & Bioelectronics 15;78:396-403.
- Gold electrodes: Introduction of amino groups by reaction with Cysteamine hydrochloride (aq) exploiting au/sh coupling; washing; covalent attachment of citrate-capped nanoparticles by formation of an amide bond; washing; immobilization of protein or antibody by covalent coupling by formation of amide bonds. See also Raghav R, Srivastava S, 2015. Core-shell gold-silver nanoparticles based impedimetric immunosensor for cancer antigen CA125. Sensors and Actuators B: Chemical 220:557-564.

Although the above procedures are directed to carbon and gold electrodes, other types of electrodes may also be used with similar procedures.

In some embodiments, the sensor according to the present disclosure is configured such that the electrode can be coupled to a benchtop, handheld electrochemical workstation, a surface plasmon resonance detector or a measurement circuit.

In some embodiments, the sensor according to the present disclosure comprises a substrate, the substrate comprising at least 3 electrodes, wherein said electrodes are configured such that they can be coupled to an electrochemical workstation.

In some embodiments, the sensor according to the present disclosure comprises a substrate, the substrate comprising at least 3 chips and wherein said chips are configured such that they can be coupled to a surface plasmon resonance detector.

In one embodiment, the sensor is configured for detection and/or quantification of a thyroid hormone.

### Methods

The present disclosure relates to a sensor for detection and/or quantification of a thyroid hormone, the sensor comprising a iodothyronine deiodinase [EC 1.21.99.3 and/or EC 1.21.99.4] or a fragment thereof, wherein the iodothyronine deiodinase is immobilized on the sensor, and uses of said sensor for diagnosis and/or monitoring of thyroid related disorders.

The present disclosure relates to a sensor for detection and/or quantification of a thyroid hormone, the sensor comprising a substrate, a first iodothyronine deiodinase selected from EC 1.21.99.4, a second iodothyronine deiodinase selected from 1.21.99.3 and EC 1.21.99.4, and an anti-rT3 antibody, wherein the iodothyronine deiodinases and the anti-rT3 antibody are immobilized on a surface of the substrate, and uses of said sensor for diagnosis and/or monitoring of thyroid related disorders.

It is an aspect of the disclosure to provide an in-vitro method for diagnosis of a thyroid related disorder in a subject comprising the steps of:
a) Providing a sample obtained from the subject,
b) Contacting the as disclosed herein with said sample,
c) Detecting the one or more thyroid hormones in the sample,
d) Determining a level and/or concentration of said thyroid hormone in the sample,
thereby diagnosing one or more thyroid related disorders.

It is also an aspect of the present disclosure to provide a method for detection of a thyroid hormone in a sample, the method comprising the steps of:
a) Providing a sample comprising or suspected of comprising a thyroid hormone,
b) Contacting the sensor disclosed herein with said sample,
c) Measuring a signal from the sensor,
thereby detecting the thyroid hormone.

It is also an aspect of the present disclosure to provide a method for quantification of a thyroid hormone in a sample, the method comprising the steps of:
a. Providing a sample comprising or suspected of comprising a thyroid hormone,
b. Contacting the sensor disclosed herein with said sample,
c. Measuring a signal from the sensor, and
d. Using the signal to determine a level and/or concentration of the one or more thyroid hormones in the sample
thereby detecting the thyroid hormone.

In a particular embodiment, the method for detection of a thyroid hormone in a sample according to the present disclosure further comprises step d) using the signal to determine a concentration of the one or more thyroid hormones in the sample.

In a particular embodiment, the methods according to the present disclosure further comprise the step of using the concentration of the thyroid hormone in the sample to calculate the *in vivo* concentration of the thyroid hormone.

In some embodiments of the method according to the present disclosure, the concentration of the thyroid hormone in the sample is determined from the reaction kinetics between said thyroid hormone and the iodothyronine deiodinase.

In particular embodiments, the concentration of the thyroid hormone is determined after the subject has received a medicament comprising a thyroid-stimulating compound.

In particular embodiments, the time after the subject has received the medicament is between 5 minutes and 48 hours, such as between 5 minutes and 45 hours, such as between 5 minutes and 40 hours, such as between 5 minutes and 36 hours, such as between 5 minutes and 32 hours, such as between 5 minutes and 30 hours, such as between 5 minutes and 28 hours, such as between 5 minutes and 24 hours, such as between 5 minutes and 20 hours, such as between 5 minutes and 18 hours, such as between 5 minutes and 16 hours, such as between 5 minutes and 14 hours, such as between 5 minutes and 12 hours, such as between 5 minutes and 11 hours, such as between 5 minutes and 10 hours, such as between 5 minutes and 9 hours, such as between 5 minutes and 8 hours, such as between 5 minutes and 7 hours, such as between 5 minutes and 6 hours, such as between 5 minutes and 5 hours, such as between 5 minutes and 4 hours, such as between 5 minutes and 3 hours, such as between 5 minutes and 2 hours, such as between 5 minutes and 1 hour, such as between 5 minutes and 45 minutes, such as between 5 minutes and 30 minutes.

In particular embodiments, the time after the subject has received the medicament is between 5 minutes and 48 hours, such as between 15 minutes and 48 hours, such as between 30 minutes and 48 hours, such as between 45 minutes and 48 hours, such as between 60 minutes and 48 hours, such as between 1 and 48 hours, such as between 2 and 48 hours, such as between 3 and 48 hours, such as between 4 and 48 hours, such as between 5 and 48 hours, such as between 6 and 48 hours, such as between 6 and 48 hours, such as between 7 and 48 hours, such as between 8 and 48 hours, such as between 9 and 48 hours, such as between 10 and 48 hours, such as between 11 and 48 hours, such as between 12 and 48 hours, such as between 14 and 48 hours, such as between 16 and 48 hours, such as between 18 and 48 hours, such as between 20 and 48 hours, such as between 24 and 48 hours, such as between 28 and 48 hours, such as between 32 and 48 hours, such as between 36 and 48 hours, such as between 40 and 48 hours, such as between 44 and 48 hours,

In one embodiment of the method according to the present disclosure, the subject has not received a medicament comprising a thyroid-stimulated hormone prior to determining the concentration of the thyroid hormone.

In particular embodiments, the method according to the present disclosure further comprises the step of comparing the level and/or concentration of said thyroid hormone in the sample with a cut-off interval to diagnose a subject of a thyroid related disorder, wherein said cut-off interval is determined from the concentration range of a thyroid hormone in healthy human individuals, such as human individuals not suffering from the thyroid related disorder,
wherein the level and/or concentration that is outside the cut-off interval indicates the presence of said thyroid related disorder.

In some embodiments of the method according to the present disclosure, the cut-off interval for free T3 is from 2.8 to 4.4 pg/mL, the cut-off interval for free T4 is from 0.8 to 2.0 ng/mL, and the cut-off interval for rT3 is from 10 to 24 ng/mL. In some embodiments, a concentration below the cut-off interval is considered low, a concentration inside the cut-off interval is considered normal, and a concentration above the cut-off interval is considered high.

In some embodiments of the method according to the present disclosure, the cut-off interval for free T3 is from 2.4 to 4.2 pg/mL, the cut-off interval for free T4 is from 0.8 to 1.8 ng/mL, and the cut-off interval for rT3 is from 10 to 24 ng/mL. In some embodiments, a concentration below the cut-off interval is considered low, a concentration inside the cut-off interval is considered normal, and a concentration above the cut-off interval is considered high.

In some embodiments of the method according to the present disclosure, the cut-off interval for free T3 is from 2.8 to 4.0 pg/mL, the cut-off interval for free T4 is from 0.8 to 2.2 ng/mL, and the cut-off interval for rT3 is from 10 to 24 ng/mL. In some embodiments, a concentration below the cut-off interval is considered low, a concentration inside the cut-off interval is considered normal, and a concentration above the cut-off interval is considered high.

In one embodiment, the method according to the present disclosure further comprises a step of treating said thyroid related disorder. In a particular embodiment, the treatment comprises administration of a medicament in a therapeutically effective amount. In a further embodiment, said medicament is a thyroid-stimulating compound.

In some embodiments, the thyroid-stimulating compound is selected from a group consisting of T3, T4, TSH, thyroid autoantibodies (TRAb, TPOAb and TgAb) and thyroglobulin.

### Subjects

It is an aspect of the disclosure to provide a method according to the present disclosure, wherein the subject is a human subject. In particular embodiments, the human subject is a child or an adult.

In further embodiments of the method according to the present disclosure, the subject is a horse, cow, sheep, pig, goat, cat or dog.

### Sample

In particular embodiments of the method according to the present disclosure, the sample is a blood sample, a serum sample or a plasma sample, optionally wherein the sample has been treated prior to analysis.

In particular embodiments, the treatment prior to analysis comprises filtering, removal of rT3 and/or adjusting pH. It is understood by the person skilled in the art that filtering of samples, such as blood samples, may provide means to remove blood cells. Adjustment of pH may be performed by the addition of a suitable acid or base to the sample until a desired pH is obtained. Suitable acids and bases for adjustment of pH are known to the person skilled in the art.

### Detection technologies

In some embodiments of the methods according to the present disclosure, the thyroid hormone is detected using surface plasmon resonance (SPR). In particular embodiments, the surface plasmon resonance readout is used to determine the concentration of one or more of the thyroid hormones.

Surface plasmon resonance is the resonant oscillation of conduction electrons at the interface between negative and positive permittivity material stimulated by incident light. SPR is the basis of many standard tools for measuring adsorption of material onto planar metal (such as gold or silver) surfaces or onto the surface of metal nanoparticles. It is the fundamental principle behind many color-based biosensor applications, different sensors and diatom photosynthesis. SPR may be used to detect biomolecular binding interactions. In SPR, one molecular partner such as a protein is immobilized on a metallic film. Light excites surface plasmons in the metal; when the binding partner binds to the immobilized molecule, this causes a detectable change in the surface plasmon signal.

In some embodiments of the methods according to the present disclosure, the iodothyronine deiodinase is immobilized on a metal surface or a layer of nanoparticles, on a substrate. In some embodiments, said substrate is a glass chip.

In some embodiments of the methods according to the present disclosure, the thyroid hormone is detected or monitored by electrochemical transduction.

Electrochemical biosensors, also referred to as biosensors utilizing electrochemical transduction provide an attractive means to analyze the content of a biological sample due to the direct conversion of a biological event to an electronic signal. The most common techniques in electrochemical biosensing comprise cyclic voltammetry, chronoamperometry, chronopotentiometry, impedance spectroscopy, and field-effect transistor based methods along with nanowire or magnetic nanoparticle-based biosensing. Additional measurement techniques useful in combination with electrochemical detection may further comprise the electrochemical versions of surface plasmon resonance, optical waveguide lightmode spectroscopy, ellipsometry, quartz crystal microbalance, and scanning probe microscopy.

The electrochemical transduction and the general performance of electrochemical sensors are often determined by the surface architectures that connect the electrode to the biological sample at the nanometer scale. The electrode surface modifications, the various electrochemical transduction mechanisms, and the choice of the biological element bound to the electrode all influence the ultimate sensitivity of the sensor.

### Thyroid related disorders

In some embodiments, the thyroid related disorder is selected from the list; hypothyroidism, hyperthyroidism, clinical depression, Goitre, Graves-Basedow disease, Hashimoto's thyroiditis, euthyroid sickness and Polar T3 syndrome.

In particular embodiments, the hyperthyroidism is characterized by high free T4, high free T3 and low TSH. In one embodiment, the euthyroid sickness is characterized by low free T3 and high rT3. In a further embodiment, the hypothyroidism is primary or secondary. In one embodiment, the primary hypothyroidism is characterized by low free T4, normal or low free T3, and high TSH. In one embodiment, the secondary hypothyroidism is characterized by low free T4, normal or low free T3, and normal or low TSH.

### Home device

It is a further aspect of the present to provide a hand-held device for detection, quantification and/or monitoring of a thyroid hormone, wherein the thyroid hormone is selected from the groups consisting of fT3, fT4 and rT3, the device comprising:
a. An inlet for a sample;
b. A sensor comprising:
   i. a substrate, and
   ii. a combination of at least 2 iodothyronine deiodinases and an anti-rT3 antibody, wherein the combination comprises:
      - type 1 iodothyronine deiodinase, type 2 iodothyronine deiodinase, and anti-rT3 antibody, or
      - type 2 iodothyronine deiodinase, type 3 iodothyronine deiodinase, and anti-rT3 antibody;
c. A detector configured to receive a signal from the sensor and transform it into a format readable by a user;
d. Optionally, means for separating cellular components from the sample.

In particular embodiments, the hand-held device according to the present disclosure comprises the sensor as defined in any one of the embodiments of the present disclosure.

### Examples

### Example 1. Estimation of T4 using IDII Amperometric Biosensor

IDII was extracted from rat brain as crude microsomal fraction, and was used to fabricate an amperometric biosensor.

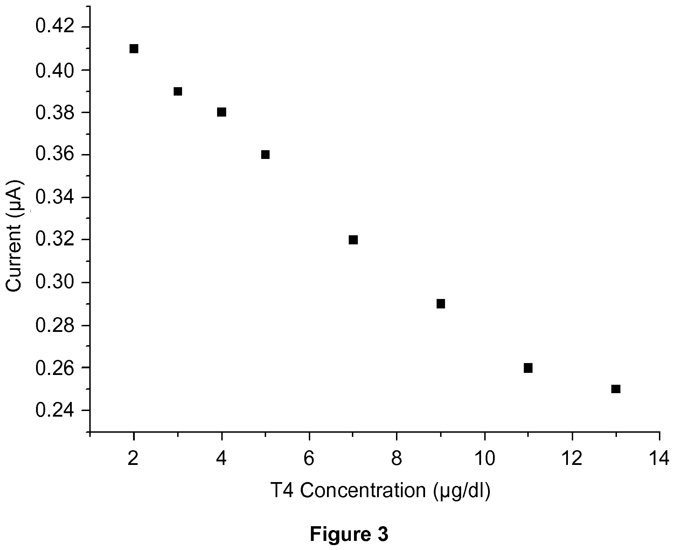

CH604E Electrochemical Analyzer/Workstation (CH instruments) has been employed to make the electrochemical biosensor. The carbon electrodes were amino-functionalized with 10uL (3-Aminopropyl)triethoxysilane (5mM, APTES), and incubated it for 2 hrs in darkness. The electrodes were rinsed with double distilled water to remove unbound 3-APTES followed by addition of 0.5 µg of citrate capped AuNPs. NPs surface was amino capped using 20 µg of 2mg/mL cysteamine hydrochloride with 2 hrs of incubation and washing followed by 10 µL of the cross linking agent (10% (v/v) aqueous solution of glutaraldehyde) that was then air dried. Finally, rat brain crude extract (microsomal fraction) was added onto the electrode and allowed to dry for 2 hrs at ambient temperature. Figure 3 shows the effect of concentration of T4 on the current response.

Results: A linear variation of current with increasing concentration of T4 is observed.

### Example 2. Estimation of T4 using IDII Voltammetric Biosensor

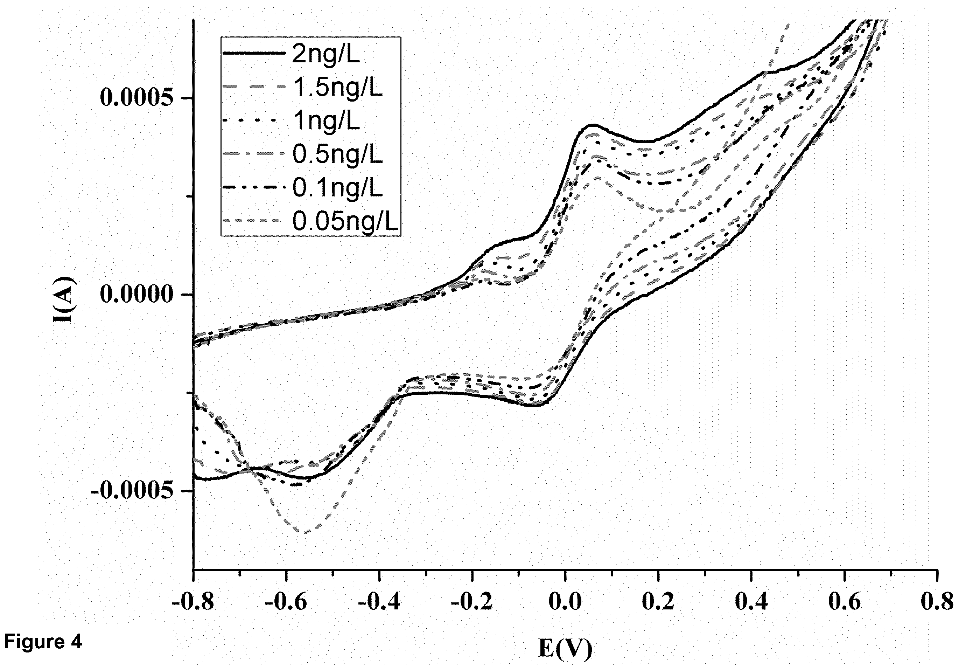

Cyclic voltammetric measurements for quantification of T4 have been shown in Figure 4.

Results: As T4 concentration increases the oxidation peak current decreases.

### Example 3. Free vs. Bound T4

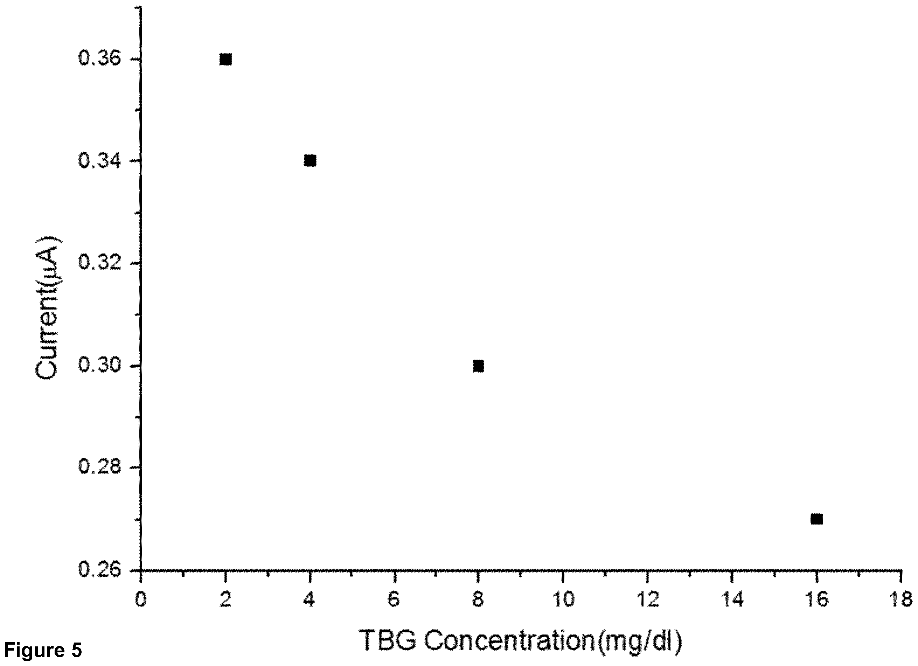

Further we studied the interference of Thyroxine-binding globulin (TBG) on detection of T4. Figure 5 shows that with increasing concentration of TBG, the current response decreases.

Results: The data demonstrate that the enzyme catalyses deiodination of only fT4 and not the bound form (tT4). Thus this strategy allows direct estimation of fT4.

### Example 4. Interference of Serum Proteins

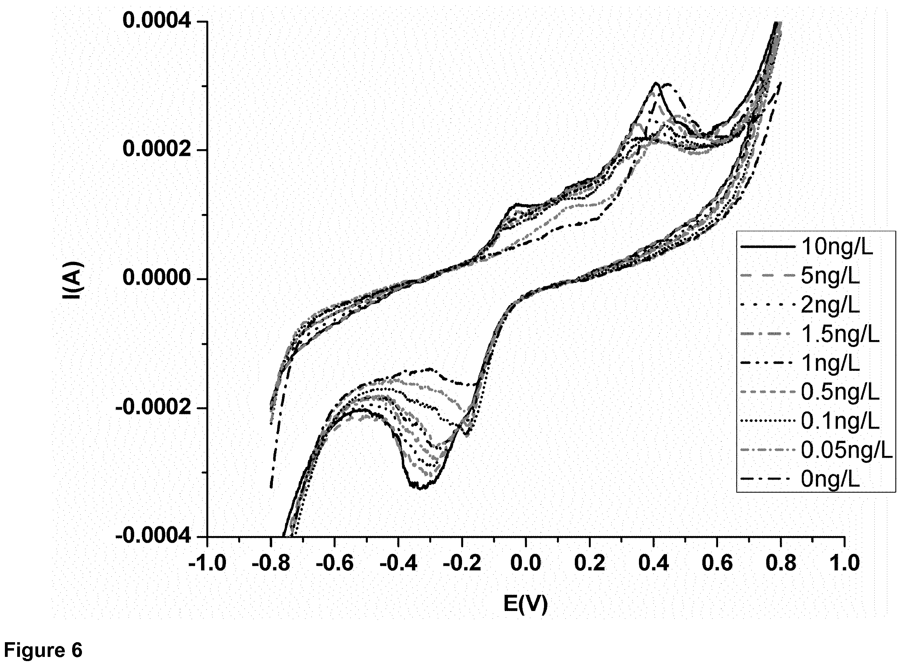

Cyclic voltammetric measurements were performed in the presence of fetal calf serum to study the interference of serum proteins in quantification. Figure 6 demonstrates these measurements in fetal calf serum with increasing T4 concentrations.

Results: The data demonstrate that the oxidation peak currents still follow a consistent trend, thereby overcoming the effect of serum proteins on the measurement.

### Example 5. Estimation of fT3 and fT4 using IDII-IDIII-anti-rT3 Voltammetric Biosensor

IDII and IDIII are extracted from rat brain as crude microsomal fraction, and are used to fabricate an amperometric biosensor.

IDII is directly linked to a surface of electrode 1, wherein said surface of electrode 1 modified with nanoparticles or wherein said surface of the electrode is modified with a layer of gold, or wherein said surface of the electrode has been roughened by other means known to the person of skills in the art.

IDIII is directly linked to a surface of electrode 2, wherein said surface of electrode 2 modified with nanoparticles or wherein said surface of the electrode is modified with a layer of gold, or wherein said surface of the electrode has been roughened by other means known to the person of skills in the art.

Anti-rT3 antibody is directly linked to a surface of electrode 3, wherein said surface of electrode 3 modified with nanoparticles or wherein said surface of the electrode is modified with a layer of gold, or wherein said surface of the electrode has been roughened by other means known to the person of skills in the art. Commercially available anti-rT3 antibody are used (for example: monoclonal anti-rT3 antibody, LifeSpan BioScience, Inc. (US); rT3 / Reverse Triiodothyronine Polyclonal Antibody, LifeSpan BioScience, Inc. (US); Anti-Reverse Triiodothyronine Antibody, MyBioSource.com (US); Reverse Triiodothyronine (rT3) Monoclonal Antibody, Biomatik (US); Reverse Triiodothyronine (rT3) Polyclonal Antibody Biomatik (US)).

CH604E Electrochemical Analyzer/Workstation (CH instruments) is employed to make the electrochemical biosensor.

DIO2 deiodinases T4 and rT3 in the sample on electrode 1, and hence measures the sum of [T4 + rT3].

DIO3 deiodinases T4 and T3 in the sample on electrode 2, and hence measures the sum of [T4 + T3].

Anti-rT3 binds to rT3, and hence measures [rT3].

Based on the obtained [T4 + rT3], [T4 + T3] and [rT3], it is possible to mathematically determine [T4] and [T3].

## Claims

1. A sensor for quantification of a thyroid hormone, the sensor comprising:
a. a substrate, and
b. a combination of at least two iodothyronine deiodinases and an anti-rT3 antibody, immobilized on a surface of the substrate, wherein the combination comprises:
• type 1 iodothyronine deiodinase, type 2 iodothyronine deiodinase, and anti-rT3 antibody, or
• type 2 iodothyronine deiodinase, type 3 iodothyronine deiodinase, and anti-rT3 antibody.

2. The sensor according to any one of the preceding claims, wherein the substrate comprises multiple surfaces for immobilization of the at least 2 iodothyronine deiodinases, such as a first surface of the substrate and a second surface of the substrate; and/or
wherein a first iodothyronine deiodinase is immobilized on the first surface of the substrate, and a second iodothyronine deiodinase is immobilized on the second surface of the substrate; and/or
wherein the anti-rT3 antibody is immobilized on a third surface of the substrate.

3. The sensor according to any one of the preceding claims, wherein the thyroid hormone is selected from free T4, free T3, reverse T3 (rT3), and combinations thereof.

4. The sensor according to any one of the preceding claims, wherein the substrate is one or more electrodes and/or chips; and/or
wherein the substrate comprises at least 1 electrode, such as at least 2 electrodes, such as at least 3 electrodes; and/or
wherein the substrate comprises at least 1 chip, such as at least 2 chips, such as at least 3 chips.

5. The sensor according to any one of the preceding claims, wherein the substrate comprises or consists of 3 electrodes, and wherein the first surface is a surface of a first electrode, the second surface is a surface of a second electrode, and the third surface is a surface of a third electrode; and/or
wherein the substrate comprises or consists of 3 chips, and wherein the first surface is a surface of a first chip, the second surface is a surface of a second chip, and the third surface is a surface of a third chip.

6. The sensor according to any one of the preceding claims, wherein the electrode is made of carbon, gold or platinum; and/or
wherein the chip is a glass chip.

7. The sensor according to any one of the preceding claims, wherein the first, second and/or third surface of the substrate is a modified surface; and/or
wherein the modified surface is a surface comprising a plurality of topographic features in the nanometre and/or micrometre size; and/or
wherein the plurality of topographic features in the nanometre and/or micrometre size are selected from the group consisting of: microparticles, nanoparticles, microwires, nanowires, microtubes, nanotubes, microrods, nanorods, and combinations thereof; and/or
wherein the modified surface is a surface coated with a layer of gold; and/or wherein the modified surface is a surface modified with nanoparticles selected from the group consisting of gold, silver, copper oxide, graphene, iron oxide and combinations thereof; and/or
wherein the modified surface is a surface coated with a layer of gold, and wherein said surface is further modified with nanoparticles selected from the group consisting of gold, silver, copper oxide, graphene, iron oxide and combinations thereof; and/or
wherein the plurality of topographic features in the nanometre and/or micrometre size have been generated on the surface of the substrate by assembling said surface by sintering, surface etching and/or particle deposition.

8. The sensor according to any one of the preceding claims, wherein the at least 2 iodothyronine deiodinases and/or the anti-rT3 antibody, are immobilized on the substrate through a linker comprising:
a. a cysteamine bound to the substrate, and
b. a nanoparticle bound to the cysteamine and to the iodothyronine deiodinase, optionally through one or more additional cysteamines.

9. The sensor according to any one of the preceding claims, configured such that the substrate can be coupled to a benchtop, handheld electrochemical workstation, a surface plasmon resonance detector or a measurement circuit; and/or
wherein the substrate comprises at least 3 electrodes and wherein said electrodes are configured such that they can be coupled to an electrochemical workstation; and/or
wherein the substrate comprises at least 3 chips and wherein said chips are configured such that they can be coupled to a surface plasmon resonance detector.

10. A method for quantification of a thyroid hormone in a sample, the method comprising the steps of:
a. Providing a sample comprising or suspected of comprising a thyroid hormone,
b. Contacting the sensor according to any one of the preceding claims with said sample,
c. Measuring a signal from the sensor, and
d. Using the signal to determine a level and/or concentration of the one or more thyroid hormones in the sample
thereby detecting the thyroid hormone.

11. An in-vitro method for diagnosis of a thyroid related disorder in a subject comprising the steps of:
a. Providing a sample obtained from the subject,
b. Determining a level and/or concentration of said thyroid hormone in the sample using the method according to claim 10,
thereby diagnosing one or more thyroid related disorders.

12. A method for manufacturing a sensor comprising at least two iodothyronine deiodinases, the method comprising:
a. Providing a substrate,
b. Providing a combination of the at least two iodothyronine deiodinases and an anti-rT3 antibody,
c. immobilizing the iodothyronine deiodinases and the anti-rT3 antibody on a surface of the substrate,
wherein the combination comprises:
• type 1 iodothyronine deiodinase, type 2 iodothyronine deiodinase, and anti-rT3 antibody, or
• type 2 iodothyronine deiodinase, type 3 iodothyronine deiodinase, and anti-rT3 antibody,
thereby manufacturing a sensor comprising at least 2 iodothyronine deiodinases.

13. The method according to claim 12, wherein the substrate is as defined in any one of the preceding claims; and/or
wherein the at least 2 iodothyronine deiodinases are as defined in any one of the preceding claims.

14. A hand-held device for quantification and/or monitoring of a thyroid hormone, the device comprising:
a. An inlet for a sample;
b. A sensor comprising:
i. a substrate, and
ii. a combination of at least 2 iodothyronine deiodinases and an anti-rT3 antibody, wherein the combination comprises:
▪ type 1 iodothyronine deiodinase, type 2 iodothyronine deiodinase, and anti-rT3 antibody, or
▪ type 2 iodothyronine deiodinase, type 3 iodothyronine deiodinase, and anti-rT3 antibody;
c. A detector configured to receive a signal from the sensor and transform it into a format readable by a user;
d. Optionally, means for separating cellular components from the sample.

15. The hand-held device according to claim 14, wherein the sensor is as defined in any one of the preceding claims; and/or
wherein the first iodothyronine deiodinase, the second iodothyronine deiodinase and the anti-rT3 antibody are as defined in any one of the preceding claims.

## Patentansprüche

1. Sensor zur Quantifizierung eines Schilddrüsenhormons, wobei der Sensor Folgendes umfasst:
a. ein Substrat, und
b. eine Kombination aus mindestens zwei Iodothyronin-Deiodinasen und einem Anti-rT3-Antikörper, immobilisiert auf einer Oberfläche des Substrats, wobei die Kombination Folgendes umfasst:
• Iodothyronin-Deiodinase Typ 1, Iodothyronin-Deiodinase Typ 2 und Anti-rT3-Antikörper oder
• Iodothyronin-Deiodinase Typ 2, Iodothyronin-Deiodinase Typ 3 und Anti-rT3-Antikörper.

2. Sensor nach einem der vorhergehenden Ansprüche, wobei das Substrat mehrere Oberflächen zur Immobilisierung der mindestens 2 Iodothyronin-Deiodinasen umfasst, wie etwa eine erste Oberfläche des Substrats und eine zweite Oberfläche des Substrats; und/oder
wobei eine erste Iodothyronin-Deiodinase auf der ersten Oberfläche des Substrats immobilisiert ist und eine zweite Iodothyronin-Deiodinase auf der zweiten Oberfläche des Substrats immobilisiert ist; und/oder
wobei der Anti-rT3-Antikörper auf einer dritten Oberfläche des Substrats immobilisiert ist.

3. Sensor nach einem der vorhergehenden Ansprüche, wobei das Schilddrüsenhormon aus freiem T4, freiem T3, reversem T3 (rT3) und Kombinationen davon ausgewählt ist.

4. Sensor nach einem der vorhergehenden Ansprüche, wobei das Substrat eine oder mehrere Elektroden und/oder Chips ist; und/oder
wobei das Substrat mindestens 1 Elektrode umfasst, wie etwa mindestens 2 Elektroden, wie etwa mindestens 3 Elektroden; und/oder
wobei das Substrat mindestens 1 Chip umfasst, wie etwa mindestens 2 Chips, wie etwa mindestens 3 Chips.

5. Sensor nach einem der vorhergehenden Ansprüche, wobei das Substrat 3 Elektroden umfasst oder daraus besteht, und wobei die erste Oberfläche eine Oberfläche einer ersten Elektrode ist, die zweite Oberfläche eine Oberfläche einer zweiten Elektrode ist und die dritte Oberfläche eine Oberfläche einer dritten Elektrode ist; und/oder
wobei das Substrat 3 Chips umfasst oder daraus besteht, und wobei die erste Oberfläche eine Oberfläche eines ersten Chips ist, die zweite Oberfläche eine Oberfläche eines zweiten Chips ist, und die dritte Oberfläche eine Oberfläche eines dritten Chips ist.

6. Sensor nach einem der vorhergehenden Ansprüche, wobei die Elektrode aus Kohlenstoff, Gold oder Platin besteht; und/oder
wobei der Chip ein Glas-Chip ist.

7. Sensor nach einem der vorhergehenden Ansprüche, wobei die erste, zweite und/oder dritte Oberfläche des Substrats eine modifizierte Oberfläche ist; und/oder
wobei die modifizierte Oberfläche eine Oberfläche ist, die eine Vielzahl von topografischen Merkmalen in Nanometer- und/oder Mikrometergröße umfasst; und/oder
wobei die Vielzahl topografischer Merkmale in Nanometer- und/oder Mikrometergröße aus der Gruppe ausgewählt ist, die aus Folgendem besteht: Mikropartikel, Nanopartikel, Mikrodrähte, Nanodrähte, Mikroröhrchen, Nanoröhrchen, Mikrostäbchen, Nanostäbchen und Kombinationen davon; und/oder
wobei die modifizierte Oberfläche eine mit einer Goldschicht beschichtete Oberfläche ist; und/oder
wobei die modifizierte Oberfläche eine Oberfläche ist, die mit Nanopartikeln modifiziert ist, die aus der Gruppe bestehend aus Gold, Silber, Kupferoxid, Graphen, Eisenoxid und Kombinationen davon ausgewählt sind; und/oder
wobei die modifizierte Oberfläche eine mit einer Goldschicht beschichtete Oberfläche ist, und wobei die Oberfläche ferner mit Nanopartikeln modifiziert ist, die aus der Gruppe bestehend aus Gold, Silber, Kupferoxid, Graphen, Eisenoxid und Kombinationen davon ausgewählt sind; und/oder
wobei die Vielzahl topografischer Merkmale in Nanometer- und/oder Mikrometergröße auf der Oberfläche des Substrats durch Zusammenbauen der Oberfläche durch Sintern, Oberflächenätzen und/oder Partikelabscheidung erzeugt wurde.

8. Sensor nach einem der vorhergehenden Ansprüche, wobei die mindestens 2 Iodothyronin-Deiodinasen und/oder der Anti-rT3-Antikörper auf dem Substrat durch einen Linker immobilisiert sind, der Folgendes umfasst:
a. ein an das Substrat gebundenes Cysteamin und
b. ein an das Cysteamin und an die Iodothyronin-Deiodinase gebundenes Nanopartikel, wahlweise über ein oder mehrere zusätzliche Cysteamine.

9. Sensor nach einem der vorhergehenden Ansprüche, der so konfiguriert ist, dass das Substrat an eine Arbeitsbank, elektrochemische Handgerät-Arbeitsstation, einen Oberflächenplasmonresonanz-Detektor oder eine Messschaltung gekoppelt werden kann; und/oder
wobei das Substrat mindestens 3 Elektroden umfasst und wobei die Elektroden so konfiguriert sind, dass sie an eine elektrochemische Arbeitsstation gekoppelt werden können; und/oder
wobei das Substrat mindestens 3 Chips umfasst, und wobei die Chips so konfiguriert sind, dass sie an einen Oberflächenplasmonresonanz-Detektor gekoppelt werden können.

10. Verfahren zur Quantifizierung eines Schilddrüsenhormons in einer Probe, wobei das Verfahren die folgenden Schritte umfasst:
a. Bereitstellen einer Probe, die ein Schilddrüsenhormon umfasst oder von der vermutet wird, ein solches zu enthalten,
b. Inkontaktbringen des Sensors nach einem der vorhergehenden Ansprüche mit der Probe,
c. Messen eines Signals von dem Sensor und
d. Verwenden des Signals zum Bestimmen eines Spiegels und/oder einer Konzentration des einen oder der mehreren Schilddrüsenhormone in der Probe,
wodurch das Schilddrüsenhormon detektiert wird.

11. In-vitro-Verfahren zur Diagnose einer mit der Schilddrüse in Zusammenhang stehenden Erkrankung bei einem Subjekt, das die folgenden Schritte umfasst:
a. Bereitstellen einer von dem Subjekt erhaltenen Probe,
b. Bestimmen eines Spiegels und/oder einer Konzentration des Schilddrüsenhormons in der Probe unter Verwendung des Verfahrens nach Anspruch 10,
wodurch eine oder mehrere mit der Schilddrüse in Zusammenhang stehende Erkrankungen diagnostiziert werden.

12. Verfahren zum Herstellen eines Sensors, der mindestens zwei Iodothyronin-Deiodinasen umfasst, wobei das Verfahren Folgendes umfasst:
a. Bereitstellen eines Substrats,
b. Bereitstellen einer Kombination aus den mindestens zwei Iodothyronin-Deiodinasen und einem Anti-rT3-Antikörper,
c. Immobilisieren der Iodothyronin-Deiodinasen und des Anti-rT3-Antikörpers auf einer Oberfläche des Substrats,
wobei die Kombination umfasst:
• Iodothyronin-Deiodinase Typ 1, Iodothyronin-Deiodinase Typ 2 und Anti-rT3-Antikörper oder
• Iodothyronin-Deiodinase Typ 2, Iodothyronin-Deiodinase Typ 3 und Anti-rT3-Antikörper,
wodurch ein Sensor hergestellt wird, der mindestens 2 Iodothyronin-Deiodinasen umfasst.

13. Verfahren nach Anspruch 12, wobei das Substrat wie in einem der vorhergehenden Ansprüche definiert ist; und/oder
wobei die mindestens 2 Iodothyronin-Deiodinasen wie in einem der vorhergehenden Ansprüche definiert sind.

14. Handgerät zur Quantifizierung und/oder Überwachung eines Schilddrüsenhormons, wobei das Gerät Folgendes umfasst:
a. einen Einlass für eine Probe;
b. einen Sensor, umfassend:
i. ein Substrat und
ii. eine Kombination aus mindestens 2 Iodothyronin-Deiodinasen und einem Anti-rT3-Antikörper, wobei die Kombination Folgendes umfasst:
▪ Iodothyronin-Deiodinase Typ 1, Iodothyronin-Deiodinase Typ 2 und Anti-rT3-Antikörper oder
▪ Iodothyronin-Deiodinase Typ 2, Iodothyronin-Deiodinase Typ 3 und Anti-rT3-Antikörper;
c. einen Detektor, der dazu konfiguriert ist, ein Signal von dem Sensor zu empfangen und es in ein von einem Benutzer lesbares Format umzuwandeln;
d. wahlweise Mittel zum Abtrennen zellulärer Komponenten aus der Probe.

15. Handgerät nach Anspruch 14, wobei der Sensor wie in einem der vorhergehenden Ansprüche definiert ist; und/oder
wobei die erste Iodothyronin-Deiodinase, die zweite Iodothyronin-Deiodinase und der Anti-rT3-Antikörper wie in einem der vorhergehenden Ansprüche definiert sind.

## Revendications

1. Capteur pour la quantification d'une hormone thyroïdienne, le capteur comprenant :
a. un substrat, et
b. une combinaison d'au moins deux iodothyronine désiodases et d'un anticorps anti-rT3, immobilisés sur une surface du substrat, dans lequel la combinaison comprend :
• une iodothyronine désiodase de type 1, une iodothyronine désiodase de type 2 et un anticorps anti-rT3, ou
• une iodothyronine désiodase de type 2, une iodothyronine désiodase de type 3 et un anticorps anti-rT3.

2. Capteur selon l'une quelconque des revendications précédentes, dans lequel le substrat comprend de multiples surfaces pour l'immobilisation des au moins 2 iodothyronine désiodases, telles qu'une première surface du substrat et une deuxième surface du substrat ; et/ou
dans lequel une première iodothyronine désiodase est immobilisée sur la première surface du substrat, et une seconde iodothyronine désiodase est immobilisée sur la deuxième surface du substrat ; et/ou
dans lequel l'anticorps anti-rT3 est immobilisé sur une troisième surface du substrat.

3. Capteur selon l'une quelconque des revendications précédentes, dans lequel l'hormone thyroïdienne est choisie parmi la T4 libre, la T3 libre, la T3 inverse (rT3), et des combinaisons de celles-ci.

4. Capteur selon l'une quelconque des revendications précédentes, dans lequel le substrat est une ou plusieurs électrodes et/ou puces ; et/ou
dans lequel le substrat comprend au moins 1 électrode, tel qu'au moins 2 électrodes, tel qu'au moins 3 électrodes ; et/ou
dans lequel le substrat comprend au moins 1 puce, tel qu'au moins 2 puces, tel qu'au moins 3 puces.

5. Capteur selon l'une quelconque des revendications précédentes, dans lequel le substrat comprend ou est constitué de 3 électrodes, et dans lequel la première surface est une surface d'une première électrode, la deuxième surface est une surface d'une deuxième électrode, et la troisième surface est une surface d'une troisième électrode ; et/ou
dans lequel le substrat comprend ou est constitué de 3 puces, et dans lequel la première surface est une surface d'une première puce, la deuxième surface est une surface d'une deuxième puce, et la troisième surface est une surface d'une troisième puce.

6. Capteur selon l'une quelconque des revendications précédentes, dans lequel l'électrode est faite de carbone, d'or ou de platine ; et/ou
dans lequel la puce est une puce en verre.

7. Capteur selon l'une quelconque des revendications précédentes, dans lequel la première, la deuxième et/ou la troisième surface du substrat est une surface modifiée ; et/ou
dans lequel la surface modifiée est une surface comprenant une pluralité de caractéristiques topographiques de l'ordre du nanomètre et/ou du micromètre ; et/ou
dans lequel la pluralité de caractéristiques topographiques de l'ordre du nanomètre et/ou du micromètre sont choisies dans le groupe constitué de : microparticules, nanoparticules, microfils, nanofils, microtubes, nanotubes, microtiges, nanotiges, et des combinaisons de ceux-ci ; et/ou
dans lequel la surface modifiée est une surface revêtue d'une couche d'or ; et/ou
dans lequel la surface modifiée est une surface modifiée par des nanoparticules choisies dans le groupe constitué de l'or, de l'argent, de l'oxyde de cuivre, du graphène, de l'oxyde de fer et des combinaisons de ceux-ci ; et/ou
dans lequel la surface modifiée est une surface revêtue d'une couche d'or, et dans lequel ladite surface est en outre modifiée par des nanoparticules choisies dans le groupe constitué de l'or, de l'argent, de l'oxyde de cuivre, du graphène, de l'oxyde de fer et des combinaisons de ceux-ci ; et/ou
dans lequel la pluralité de caractéristiques topographiques de l'ordre du nanomètre et/ou du micromètre ont été générées sur la surface du substrat par assemblage de ladite surface par frittage, gravure de surface et/ou dépôt de particules.

8. Capteur selon l'une quelconque des revendications précédentes, dans lequel les au moins 2 iodothyronine désiodases et/ou l'anticorps anti-rT3 sont immobilisés sur le substrat par l'intermédiaire d'un lieur comprenant :
a. une cystéamine liée au substrat, et
b. une nanoparticule liée à la cystéamine et à l'iodothyronine désiodase, optionnellement par l'intermédiaire d'une ou plusieurs cystéamines supplémentaires.

9. Capteur selon l'une quelconque des revendications précédentes, configuré de telle manière que le substrat peut être couplé à une paillasse, une station de travail électrochimique mobile, un détecteur de résonance plasmonique de surface ou un circuit de mesure ; et/ou
dans lequel le substrat comprend au moins 3 électrodes et dans lequel lesdites électrodes sont configurées de telle manière qu'elles peuvent être couplées à une station de travail électrochimique ; et/ou
dans lequel le substrat comprend au moins 3 puces et dans lequel lesdites puces sont configurées de telle manière qu'elles peuvent être couplées à un détecteur de résonance plasmonique de surface.

10. Procédé de quantification d'une hormone thyroïdienne dans un échantillon, le procédé comprenant les étapes de :
a. Fourniture d'un échantillon comprenant ou soupçonné de comprendre une hormone thyroïdienne,
b. Mise en contact du capteur selon l'une quelconque des revendications précédentes avec ledit échantillon,
c. Mesure d'un signal à partir du capteur, et
d. Utilisation du signal pour déterminer un niveau et/ou une concentration des une ou plusieurs hormones thyroïdiennes dans l'échantillon
pour ainsi détecter l'hormone thyroïdienne.

11. Procédé in vitro de diagnostic d'un trouble associé à la thyroïde chez un sujet comprenant les étapes de :
a. Fourniture d'un échantillon obtenu à partir du sujet,
b. Détermination d'un niveau et/ou d'une concentration de ladite hormone thyroïdienne dans l'échantillon en utilisant le procédé selon la revendication 10,
pour ainsi diagnostiquer un ou plusieurs troubles associés à la thyroïde.

12. Procédé de fabrication d'un capteur comprenant au moins deux iodothyronine désiodases, le procédé comprenant :
a. La fourniture d'un substrat,
b. La fourniture d'une combinaison des au moins deux iodothyronine désiodases et d'un anticorps anti-rT3,
c. l'immobilisation des iodothyronine désiodases et de l'anticorps anti-rT3 sur une surface du substrat,
dans lequel la combinaison comprend :
• une iodothyronine désiodase de type 1, une iodothyronine désiodase de type 2 et un anticorps anti-rT3, ou
• une iodothyronine désiodase de type 2, une iodothyronine désiodase de type 3 et un anticorps anti-rT3,
pour ainsi fabriquer un capteur comprenant au moins 2 iodothyronine désiodases.

13. Procédé selon la revendication 12, dans lequel le substrat est tel que défini dans l'une quelconque des revendications précédentes ; et/ou
dans lequel les au moins 2 iodothyronine désiodases sont telles que définies dans l'une quelconque des revendications précédentes.

14. Dispositif mobile de quantification et/ou de surveillance d'une hormone thyroïdienne, le dispositif comprenant :
a. Une entrée pour un échantillon ;
b. Un capteur comprenant :
i. un substrat, et
ii. une combinaison d'au moins 2 iodothyronine désiodases et d'un anticorps anti-rT3, dans lequel la combinaison comprend :
▪ une iodothyronine désiodase de type 1, une iodothyronine désiodase de type 2 et un anticorps anti-rT3, or
▪ une iodothyronine désiodase de type 2, une iodothyronine désiodase de type 3 et un anticorps anti-rT3 ;
c. Un détecteur configuré pour recevoir un signal à partir du capteur et le transformer en un format lisible par un utilisateur ;
d. Facultativement, un moyen de séparation de composants cellulaires à partir de l'échantillon.

15. Dispositif mobile selon la revendication 14, dans lequel le capteur est selon l'une quelconque des revendications précédentes ; et/ou
dans lequel la première iodothyronine désiodase, la seconde iodothyronine désiodase et l'anticorps anti-rT3 sont tels que définis dans l'une quelconque des revendications précédentes.


### SEQUENCE LISTING

<110> Thyrolytics AB
<120> Biosensor for Diagnosis of Thyroid Dysfunction
<130> P4912PC00
<150> EP 18183439.1
   <151> 2018-07-13
<160> 3
<170> PatentIn version 3.5
<210> 1
   <211> 249
   <212> PRT
   <213> Homo sapiens
<220>
   <221> PEPTIDE
   <222> (1)..(1)
   <223> DIO1
<220>
   <221> PEPTIDE
   <222> (126)..(126)
   <223> X=Selenocystein
<400> 1
   Met Gly Leu Pro Gln Pro Gly Leu Trp Leu Lys Arg Leu Trp Val Leu
   1               5                   10                  15
   Leu Glu Val Ala Val His Val Val Val Gly Lys Val Leu Leu Ile Leu
               20                  25                  30
   Phe Pro Asp Arg Val Lys Arg Asn Ile Leu Ala Met Gly Glu Lys Thr
           35                  40                  45
   Gly Met Thr Arg Asn Pro His Phe Ser His Asp Asn Trp Ile Pro Thr
       50                  55                  60
   Phe Phe Ser Thr Gln Tyr Phe Trp Phe Val Leu Lys Val Arg Trp Gln
   65                  70                  75                  80
   Arg Leu Glu Asp Thr Thr Glu Leu Gly Gly Leu Ala Pro Asn Cys Pro
                   85                  90                  95
   Val Val Arg Leu Ser Gly Gln Arg Cys Asn Ile Trp Glu Phe Met Gln
               100                 105                 110
   Gly Asn Arg Pro Leu Val Leu Asn Phe Gly Ser Cys Thr Xaa Pro Ser
           115                 120                 125
   Phe Met Phe Lys Phe Asp Gln Phe Lys Arg Leu Ile Glu Asp Phe Ser
   130 135 140
   Ser Ile Ala Asp Phe Leu Val Ile Tyr Ile Glu Glu Ala His Ala Ser
   145 150 155 160
   Asp Gly Trp Ala Phe Lys Asn Asn Met Asp Ile Arg Asn His Gln Asn
   165 170 175
   Leu Gln Asp Arg Leu Gln Ala Ala His Leu Leu Leu Ala Arg Ser Pro
   180 185 190
   Gln Cys Pro Val Val Val Asp Thr Met Gln Asn Gln Ser Ser Gln Leu
   195 200 205
   Tyr Ala Ala Leu Pro Glu Arg Leu Tyr Ile Ile Gln Glu Gly Arg Ile
   210 215 220
   Leu Tyr Lys Gly Lys Ser Gly Pro Trp Asn Tyr Asn Pro Glu Glu Val
   225 230 235 240
   Arg Ala Val Leu Glu Lys Leu His Ser
   245
<210> 2
   <211> 273
   <212> PRT
   <213> Homo sapiens
<220>
   <221> PEPTIDE
   <222> (1)..(1)
   <223> DIO2
<220>
   <221> PEPTIDE
   <222> (133)..(133)
   <223> X=selenocysteine
<220>
   <221> PEPTIDE
   <222> (266)..(266)
   <223> X=selenocysteine
<400> 2
   Met Gly Ile Leu Ser Val Asp Leu Leu Ile Thr Leu Gln Ile Leu Pro
   1 5 10 15
   Val Phe Phe Ser Asn Cys Leu Phe Leu Ala Leu Tyr Asp Ser Val Ile
   20 25 30
   Leu Leu Lys His Val Val Leu Leu Leu Ser Arg Ser Lys Ser Thr Arg
           35                  40                  45
   Gly Glu Trp Arg Arg Met Leu Thr Ser Glu Gly Leu Arg Cys Val Trp
       50                  55                  60
   Lys Ser Phe Leu Leu Asp Ala Tyr Lys Gln Val Lys Leu Gly Glu Asp
   65                  70                  75                  80
   Ala Pro Asn Ser Ser Val Val His Val Ser Ser Thr Glu Gly Gly Asp
                   85                  90                  95
   Asn Ser Gly Asn Gly Thr Gln Glu Lys Ile Ala Glu Gly Ala Thr Cys
               100                 105                 110
   His Leu Leu Asp Phe Ala Ser Pro Glu Arg Pro Leu Val Val Asn Phe
           115                 120                 125
   Gly Ser Ala Thr Xaa Pro Pro Phe Thr Ser Gln Leu Pro Ala Phe Arg
       130                 135                 140
   Lys Leu Val Glu Glu Phe Ser Ser Val Ala Asp Phe Leu Leu Val Tyr
   145                 150                 155                 160
   Ile Asp Glu Ala His Pro Ser Asp Gly Trp Ala Ile Pro Gly Asp Ser
                   165                 170                 175
   Ser Leu Ser Phe Glu Val Lys Lys His Gln Asn Gln Glu Asp Arg Cys
               180                 185                 190
   Ala Ala Ala Gln Gln Leu Leu Glu Arg Phe Ser Leu Pro Pro Gln Cys
           195                 200                 205
   Arg Val Val Ala Asp Arg Met Asp Asn Asn Ala Asn Ile Ala Tyr Gly
       210                 215                 220
   Val Ala Phe Glu Arg Val Cys Ile Val Gln Arg Gln Lys Ile Ala Tyr
   225                 230                 235                 240
   Leu Gly Gly Lys Gly Pro Phe Ser Tyr Asn Leu Gln Glu Val Arg His
                   245                 250                 255
   Trp Leu Glu Lys Asn Phe Ser Lys Arg Xaa Lys Lys Thr Arg Leu Ala
               260                 265                 270
   Gly
<210> 3
   <211> 304
   <212> PRT
   <213> Homo sapiens
<220>
   <221> PEPTIDE
   <222> (1)..(1)
   <223> DIO3
<220>
   <221> PEPTIDE
   <222> (170)..(170)
   <223> X=selenocysteine
<400> 3
   Met Pro Arg Gln Ala Thr Ser Arg Leu Val Val Gly Glu Gly Glu Gly
   1               5                   10                  15
   Ser Gln Gly Ala Ser Gly Pro Ala Ala Thr Met Leu Arg Ser Leu Leu
               20                  25                  30
   Leu His Ser Leu Arg Leu Cys Ala Gln Thr Ala Ser Cys Leu Val Leu
           35                  40                  45
   Phe Pro Arg Phe Leu Gly Thr Ala Phe Met Leu Trp Leu Leu Asp Phe
       50                  55                  60
   Leu Cys Ile Arg Lys His Phe Leu Gly Arg Arg Arg Arg Gly Gln Pro
   65                  70                  75                  80
   Glu Pro Glu Val Glu Leu Asn Ser Glu Gly Glu Glu Val Pro Pro Asp
                   85                  90                  95
   Asp Pro Pro Ile Cys Val Ser Asp Asp Asn Arg Leu Cys Thr Leu Ala
               100                 105                 110
   Ser Leu Lys Ala Val Trp His Gly Gln Lys Leu Asp Phe Phe Lys Gln
           115                 120                 125
   Ala His Glu Gly Gly Pro Ala Pro Asn Ser Glu Val Val Leu Pro Asp
       130                 135                 140
   Gly Phe Gln Ser Gln His Ile Leu Asp Tyr Ala Gln Gly Asn Arg Pro
   145                 150                 155                 160
   Leu Val Leu Asn Phe Gly Ser Cys Thr Xaa Pro Pro Phe Met Ala Arg
                   165                 170                 175
   Met Ser Ala Phe Gln Arg Leu Val Thr Lys Tyr Gln Arg Asp Val Asp
               180                 185                 190
   Phe Leu Ile Ile Tyr Ile Glu Glu Ala His Pro Ser Asp Gly Trp Val
           195                 200                 205
   Thr Thr Asp Ser Pro Tyr Ile Ile Pro Gln His Arg Ser Leu Glu Asp
       210                 215                 220
   Arg Val Ser Ala Ala Arg Val Leu Gln Gln Gly Ala Pro Gly Cys Ala
   225                 230                 235                 240
   Leu Val Leu Asp Thr Met Ala Asn Ser Ser Ser Ser Ala Tyr Gly Ala
                   245                 250                 255
   Tyr Phe Glu Arg Leu Tyr Val Ile Gln Ser Gly Thr Ile Met Tyr Gln
               260                 265                 270
   Gly Gly Arg Gly Pro Asp Gly Tyr Gln Val Ser Glu Leu Arg Thr Trp
           275                 280                 285
   Leu Glu Arg Tyr Asp Glu Gln Leu His Gly Ala Arg Pro Arg Arg Val
       290                 295                 300